# EUROPEAN PATENT APPLICATION

(11) **EP 4 533 942 A1**
(43) Date of publication of application: **09.04.2025**
(21) Application number: 23816169.9
(22) Date of filing: 02.06.2023
(51) Int. Cl.: A01K 67/027, D02G 3/02, D03D 15/233, D04B 1/14, D04B 21/00, C12N 15/09, C12N 15/12

(54) **GENETICALLY MODIFIED ANIMAL-DERIVED ANIMAL FIBER**

(30) Priority: 03.06.2022 JP 2022090657
(71) Applicant: Setsurotech Inc., Tokushima-shi, Tokushima, 770-0053 (JP)
(72) Inventor: SAWATSUBASHI, Shun, Tokushima-shi, Tokushima 770-0053 (JP); INATANI, Yuka, Tokushima-shi, Tokushima 770-0053 (JP); WATANABE, Tadayoshi, Tokushima-shi, Tokushima 770-0053 (JP); NAGAO, Yusuke, Tokushima-shi, Tokushima 770-0053 (JP); AGARI, Takahiro, Tokushima-shi, Tokushima 770-0053 (JP); KAINUMA, Risa, Tokushima-shi, Tokushima 770-0053 (JP); NAGAO, Tomoko, Tokushima-shi, Tokushima 770-0053 (JP); NISHIMURA, Koichi, Tokushima-shi, Tokushima 770-0053 (JP); HOZUMI, Shunya, Tokushima-shi, Tokushima 770-0053 (JP); ONO, Akihiro, Osaka-shi, Osaka 540-8622 (JP)
(74) Representative: Klöckner, Christoph
(86) International application number: PCT/JP2023/020690
(87) International publication number: WO 2023/234412

(57) **Abstract**

The present invention provides a non-human animal having a novel fiber and a method for preparing the non-human animal. Specifically, the present invention provides a non-human animal with reduced expression or functions of at least one gene constituting the Krt or KAP family, and a method for preparing the non-human animal.

## Description

### Technical Field

The present invention relates to a method for preparing an animal, an animal and an animal fiber obtained by the method, and a fiber product comprising the animal fiber.

### Background Art

The quality of animal fibers such as wool or cashmere depends on factors, for example, fiber diameters, fiber lengths, and color. Among them, thin fiber diameters are related directly to texture, wear comfort, sheen, heat retention, stretchiness, and the like of products such as textile or knit and are therefore very important factors that influence the quality of final products. Thin animal fibers have been produced so far using particular excellent breeds. However, even the excellent varieties have such thin animal fibers only in a very small proportion of the whole body hair. Since thinner animal fibers are scarcer, price varies easily depending on slight increase or decrease in hair yield, leading to unstable supply.

Animal fibers have a scale structure that generates asperities on fiber surface. The friction of these scales causes fiber entanglement, which is responsible for pilling (pill formation) as well as quality deterioration that impairs sheen, softness, smoothness, and the like. In order to avoid such deterioration, a chemical method for peeling scales or treatment for smoothing fiber surface by coating scale asperities with a coating resin has heretofore been developed. However, such a method impairs the original quality of animal fibers, though the method can prevent fiber entanglement. Therefore, the application thereof to animal fibers has been avoided, and the application to rare animal fibers having thin fiber diameters has tended to be particularly avoided.

Thus, there has been a demand for techniques that can stably supply animal fibers having thin fiber diameters and/or enable the maintenance of the quality thereof.

### Citation List

### Patent Literature

Patent Literature 1: Japanese Patent Laid-Open No. 2006-283254
Patent Literature 2: Japanese Patent Laid-Open No. 2006-132059

### Summary of Invention

The present invention provides a method for preparing a human animal, an animal and an animal fiber obtained by the method, and a fiber product comprising the animal fiber.

While pursuing diligent studies to solve the problems, the present inventors have found that: the diameter of an animal fiber is thinned by reducing expression or functions of a gene constituting the Krt or Kap family; and an animal fiber having a thin fiber diameter can be obtained from a non-human animal having the reduction. While pursuing further studies, the present inventors have also found that an animal fiber having smooth fiber surface can be obtained from the animal. The present invention has been completed on the basis of these findings.

Specifically, the present invention relates to the following:
[1] A genetically modified animal (preferably mammal, more preferably Artiodactyla) or an animal fiber thereof, wherein
   the animal has the animal fiber, and
   the genetic modification is reduction or deficiency in expression or functions of at least one gene constituting the Krt or KAP family, or disruption of the at least one gene.
[2] The genetically modified animal or the animal fiber thereof according to [1], wherein
   (i) the animal fiber of the genetically modified animal has a smaller major axis (e.g., a 5 to 50% or 5 to 25% smaller major axis) than that of the animal fiber of a syngeneic or allogeneic control animal that does not have the genetic modification, and/or
   (ii-1) the animal fiber of the genetically modified animal has a smaller content of a protein encoded by the at least one gene than that of the animal fiber of a syngeneic or allogeneic control animal that does not have the genetic modification, or (ii-2) the animal fiber of the genetically modified animal contains no protein encoded by the at least one gene.
[3] The genetically modified animal or the animal fiber thereof according to [1] or [2], wherein
   the animal fiber of the genetically modified animal has an at least 5%, at least 10%, or at least 15% smaller major axis than that of the animal fiber of an animal that does not have the genetic modification.
[4] The genetically modified animal or the animal fiber thereof according to any of [1] to [3], wherein
   the at least one gene constituting the Krt or KAP family comprises either or both of Kap8-1 and Kap8-2.
[5] The genetically modified animal or the animal fiber thereof according to any of [1] to [3], wherein
   the at least one gene constituting the Krt or KAP family at least comprises at least one gene selected from Krt71, Krt72, Krt73, and Krt74.
[6] The genetically modified animal or the animal fiber thereof according to any of [1] to [5], wherein the genetic modification further comprises reduction or deficiency in expression or functions of either or both of FGF-5 gene and Tyr gene, or disruption of the gene(s).
[7] The animal fiber according to any of [1] to [6].
[8] Cloth, textile, or a fiber product comprising the animal fiber according to [7].
[9] The genetically modified animal or the animal fiber thereof according to any of the above items, wherein the non-human animal is a mammal or a bird.
[10] The genetically modified animal or the animal fiber thereof according to [9], wherein the mammal is a mouse, a rat, a rabbit, a goat, sheep, an alpaca, a llama, a camel, a yak, an ibex, a guanaco, a qiviuk, a chiru, a raccoon dog, a raccoon, a mink, a sable, a fox, a possum, an opossum, a beaver, a seal, a nutria, a ferret, or a vicugna, or a mammal obtained by the crossing of any one of these mammals, and the bird is a wild duck, a goose, a duck, or an ostrich, or a bird obtained by the crossing of any one of these birds.
[11] The genetically modified animal or the animal fiber thereof (preferably, the animal fiber is underfur or undercoat) according to [10], wherein the goat is a goat selected from cashmere, mohair, Angora, and Cashgora goats (preferably a cashmere goat).
[12] The genetically modified animal or the animal fiber thereof according to any of the above items, wherein the genetically modified animal has an animal fiber having a larger (e.g., 5% to 10% larger) scale interval than that of the animal fiber of a syngeneic or allogeneic control animal that does not have the genetic modification.
[13] The genetically modified animal or the animal fiber thereof according to any of the above items, wherein the genetically modified animal has an animal fiber having a smaller (e.g., 5% to 20% smaller) scale thickness than that of the animal fiber of a syngeneic or allogeneic control animal that does not have the genetic modification.
[14] The genetically modified animal or the animal fiber thereof according to [12], wherein the genetically modified animal has an animal fiber having a smaller (e.g., 5% to 20% smaller) scale thickness than that of the animal fiber of a syngeneic or allogeneic control animal that does not have the genetic modification.
[15] The genetically modified animal or the animal fiber thereof according to any of the above items, wherein the animal fiber has sheen as compared with the animal fiber of a syngeneic or allogeneic control animal that does not have the genetic modification.
[16] The genetically modified animal or the animal fiber thereof according to [2], wherein the genetically modified animal has an animal fiber having a larger (e.g., 5% to 10% larger) scale interval than that of the animal fiber of a syngeneic or allogeneic control animal that does not have the genetic modification.
[17] The genetically modified animal or the animal fiber thereof according to [2], wherein the genetically modified animal has an animal fiber having a smaller (e.g., 5% to 20% smaller) scale thickness than that of the animal fiber of a syngeneic or allogeneic control animal that does not have the genetic modification.
[18] The genetically modified animal or the animal fiber thereof according to [16], wherein the genetically modified animal has an animal fiber having a smaller (e.g., 5% to 20% smaller) scale thickness than that of the animal fiber of a syngeneic or allogeneic control animal that does not have the genetic modification.
[19] A material for use in obtaining a genetically modified animal according to any of the above items, wherein the material comprises the genetically modified animal having a genetic modification or a germ cell (e.g., sperm or ovum) thereof, and the genetic modification is reduction or deficiency in expression or functions of at least one gene constituting the Krt or KAP family, or disruption of the at least one gene.
[20] The material according to [19], wherein
   the at least one gene constituting the Krt or KAP family comprises either or both of Kap8-1 and Kap8-2.
[21] The material according to [19], wherein
   the at least one gene constituting the Krt or KAP family at least comprises at least one gene selected from Krt71, Krt72, Krt73, and Krt74.
[22] The material according to [20], wherein
   the at least one gene constituting the Krt or KAP family at least comprises at least one gene selected from Krt71, Krt72, Krt73, and Krt74.
[23] The material according to [19], wherein the genetic modification further comprises reduction or deficiency in expression or functions of either or both of FGF-5 gene and Tyr gene, or disruption of the gene(s).
[24] The material according to [20], further comprising reduction or deficiency in expression or functions of either or both of FGF-5 gene and Tyr gene, or disruption of the gene(s).
[25] The material according to [21], further comprising reduction or deficiency in expression or functions of either or both of FGF-5 gene and Tyr gene, or disruption of the gene(s).
[26] The material according to [22], further comprising reduction or deficiency in expression or functions of either or both of FGF-5 gene and Tyr gene, or disruption of the gene(s).
[27] The genetically modified animal or the animal fiber thereof according to any of the above items, wherein the genetically modified animal is a male.
[28] The genetically modified animal or the animal fiber thereof according to any of the above items, wherein the genetically modified animal is a female.
[29] The material according to any of the above items, wherein the genetically modified animal is a male.
[30] The material according to any of the above items, wherein the genetically modified animal is a female.
[31] The raw hair of an animal fiber according to any of the above items, the raw hair comprising guard hair and vellus hair.
[32] Produced hair obtainable from the raw hair according to [31].
[33] A woolen yarn or a twisted yarn comprising the produced hair according to [32].
[34] Textile comprising the woolen yarn or the twisted yarn according to [33].
[35] Clothing comprising the textile according to [34].
[36] An animal fiber product comprising the textile according to [34].

(1) A method for preparing a non-human animal, comprising reducing expression or functions of at least one gene constituting the non-human animal Krt or KAP family.
(2) The method according to (1), wherein the at least one gene constituting the non-human animal Krt family is a gene encoding Krt protein expressed in the inner root sheath, cuticle, hair cortex, or hair medulla of the non-human animal.
(3) The method according to (2), wherein the gene encoding Krt protein expressed in the inner root sheath is at least one gene selected from Krt71, Krt72, Krt73, and Krt74.
(4) The method according to (3), wherein the gene is at least one gene selected from Krt71, Krt72, Krt73, and Krt74.
(5) The method according to any one of (1) to (4), wherein the at least one gene constituting the non-human animal KAP family comprises KAP8-1.
(6) The method according to any one of (1) to (5), further comprising reducing expression or functions of non-human animal Fgf5 and/or Tyr gene.
(7) The method according to any one of (1) to (6), wherein the reduction in expression or functions of the gene is knockout of the non-human animal gene.
(8) The method according to (7), wherein the non-human animal belongs to the order *Rodentia* or *Artiodactyla.*
(9) A non-human animal prepared by the method according to any one of (1) to (8) or progeny thereof.
(10) An animal fiber obtained from the non-human animal or progeny thereof according to (9).
(11) An animal fiber product comprising the animal fiber according to (10).

<1> A non-human animal or progeny thereof comprising modification that causes reduction in expression or functions of at least one gene constituting the Krt family or the KAP family.
<2> The non-human animal or the progeny thereof according to <1>, wherein the gene constituting the Krt family is selected from the group consisting of Krt71, Krt72, Krt73, and Krt74, and the gene constituting the KAP family is selected from the group consisting of the KAP8 subfamilies.
<3> The non-human animal or the progeny thereof according to <1> or <2>, further comprising modification that causes reduction in expression or functions of Fgf5 and/or Tyr gene.
<4> The non-human animal or the progeny thereof according to any one of <1> to <3>, wherein the modification that causes reduction in expression or functions of the gene is knockout.
<5> A method for preparing a non-human animal having a significantly thinner average fiber diameter of an animal fiber than a wild-type one, comprising reducing expression or functions of at least one gene selected from a gene constituting the non-human animal Krt family, a gene constituting the non-human animal KAP family, a gene constituting the non-human animal Krt family, and a gene constituting the non-human animal KAP family.
<6> A method for preparing a non-human animal having a significantly smaller average scale thickness of an animal fiber than a wild-type one, comprising reducing expression or functions of at least one gene constituting the non-human animal Krt family or KAP family.
<7> A method for preparing a non-human animal having a significantly longer scale interval of an animal fiber than a wild-type one, comprising reducing expression or functions of at least one gene selected from a gene constituting the non-human animal Krt family and a gene constituting the non-human animal KAP family.
<8> The method according to any one of <5> to <7>, wherein the gene constituting the Krt family is selected from the group consisting of Krt71, Krt72, Krt73, and Krt74, and the gene constituting the KAP family is selected from the group consisting of the KAP8-1 subfamilies.
<9> The method according to any one of <5> to <8>, further comprising reducing expression or functions of non-human animal Fgf5 and/or Tyr gene.
<10> The method according to any one of <5> to <9>, wherein the reduction in or loss of expression or functions of the gene is knockout of the gene.
<11> A non-human animal prepared by the method according to any one of <5> to <10> or progeny thereof.
<12> The method according to any one of <5> to <11>, wherein the non-human animal or the progeny thereof is a mammal or a bird.
<13> The method according to <12>, wherein the mammal is a mouse, a rat, a rabbit, a goat, sheep, an alpaca, a llama, a camel, a yak, an ibex, a guanaco, a qiviuk, a chiru, a raccoon dog, a raccoon, a mink, a sable, a fox, a possum, an opossum, a beaver, a seal, a nutria, a ferret, or a vicugna, or a mammal obtained by the crossing of any one of these mammals, and the bird is a wild duck, a goose, a duck, or an ostrich, or a bird obtained by the crossing of any one of these birds.
<14> The method according to <13>, wherein the goat is selected from cashmere, mohair, Angora, and Cashgora goats.
<15> A non-human animal prepared by the method according to any of <5> to <14> or progeny thereof.
<16> An animal fiber obtained from the non-human animal or progeny thereof according to any of <1> to <4> or <15>.
<17> An animal fiber product comprising the animal fiber according to <16>.

(P1) An animal fiber of a genetically modified non-human animal, wherein
   the non-human animal has the animal fiber, and
   the genetic modification is reduction or deficiency in expression or functions of at least one gene selected from the group consisting of Krt71, Krt72, Krt74, and KAP8-1, or disruption of the at least one gene, {on the proviso that the non-human animal may be neither a dog nor a bovine}.
(P2) The animal fiber according to (P1), wherein
   (1) the animal fiber of the genetically modified animal has a smaller content of a protein encoded by the at least one gene than that of the animal fiber of an animal that does not have the genetic modification, or (2) the animal fiber of the genetically modified animal contains no protein encoded by the at least one gene.
(P3) The animal fiber according to (P1) or (P2), wherein
   the animal fiber of the genetically modified animal has an at least 5%, at least 10%, or at least 15% smaller major axis than that of the animal fiber of an animal that does not have the genetic modification.
(P4) The animal fiber according to (P1) or (P2), wherein
   the at least one gene at least comprises Kap8-1.
(P5) The animal fiber according to (P3), wherein
   the at least one gene at least comprises Kap8-1.
(P6) The animal fiber according to (P1) or (P2), wherein
   the at least one gene at least comprises at least one gene selected from Krt71, Krt72, and Krt74.
(P7) The animal fiber according to (P3), wherein
   the at least one gene at least comprises at least one gene selected from Krt71, Krt72, and Krt74.
(P8) The animal fiber according to (P1) or (P2), wherein the genetic modification further comprises reduction or deficiency in expression or functions of either or both of FGF-5 gene and Tyr gene, or disruption of the gene(s).
(P9) The animal fiber according to (P4), wherein the genetic modification further comprises reduction or deficiency in expression or functions of either or both of FGF-5 gene and Tyr gene, or disruption of the gene(s).
(P10) The animal fiber according to (P6), wherein the genetic modification further comprises reduction or deficiency in expression or functions of either or both of FGF-5 gene and Tyr gene, or disruption of the gene(s).
(P11) Cloth, textile, or a fiber product comprising the animal fiber according to (P8).
(P12) Cloth, textile, or a fiber product comprising the animal fiber according to (P9).
(P13) Cloth, textile, or a fiber product comprising the animal fiber according to (P10).
(P14) Cloth, textile, or a fiber product comprising the animal fiber according to (P1) or (P2).

An animal breed having an animal fiber having a thin fiber diameter can be prepared by the method of the present invention. Furthermore, the animal fiber having a thin fiber diameter obtained from such an animal breeds less among individuals, and the whole body hair is thin. Hence, it is considered that the supply of a high-quality animal fiber excellent in texture, softness, stretchiness, heat retention, fast dryness, and the like can be achieved. Moreover, such an animal fiber has smooth fiber surface, in addition to the thin fiber diameter. Thus, such an animal fiber is superior in quality such as texture, wear comfort, and sheen to a conventional animal fiber having an equivalent fiber diameter. Such an animal fiber is unlikely to cause pilling or felting and can therefore maintain the quality over a long period. In addition, a non-conventional natural fiber having high safety can be produced due to lack of need for using various chemical methods that have been used in thinning fiber diameters and producing animal fibers having smooth surface. Thus, the present inventors have demonstrated that a natural fiber having preferable characteristics as an animal fiber can be produced by genetic modification of an animal that yields an animal fiber, and found a novel approach of fiber production that is excellent in low environmental load and safety and is different from conventional fiber production based on a chemical approach.

### Brief Description of Drawings

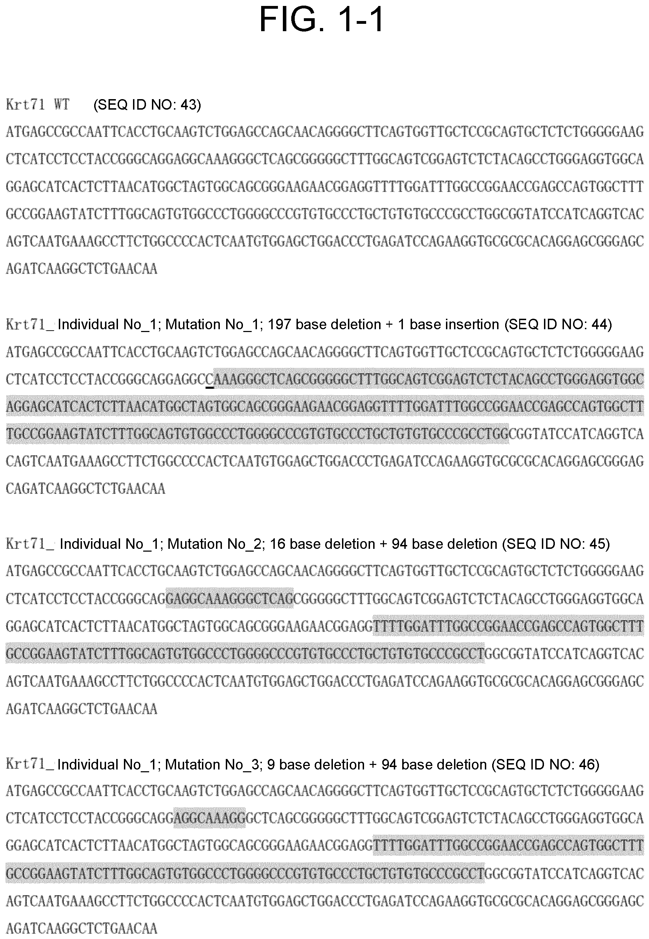

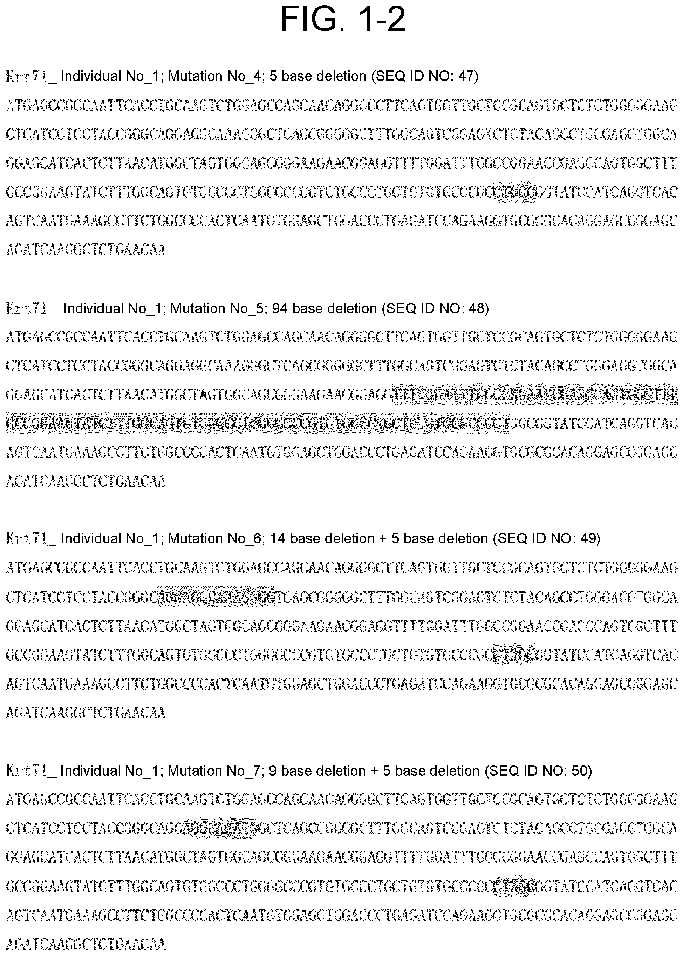

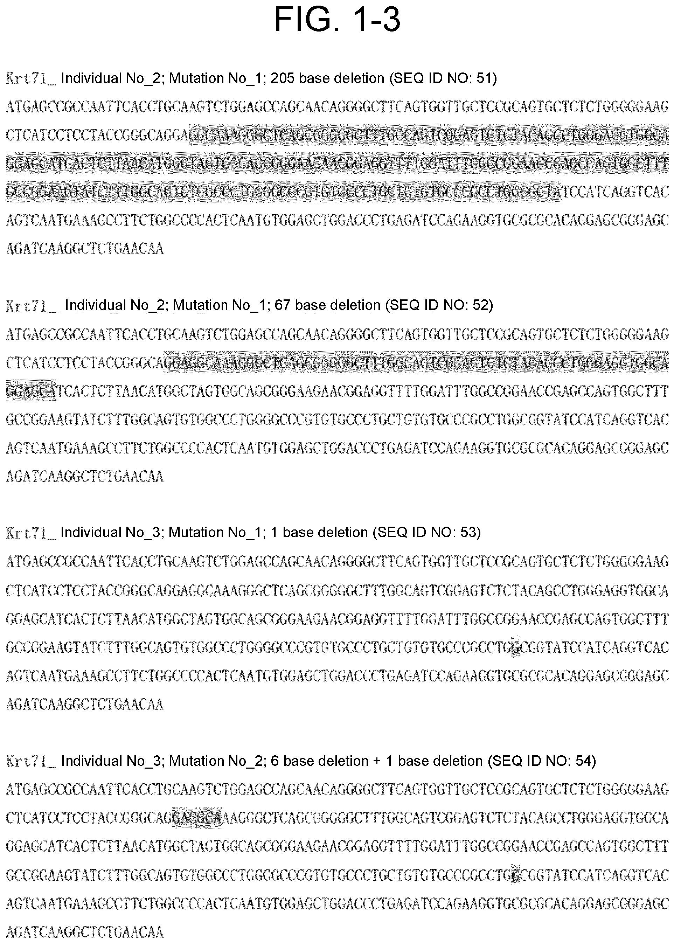

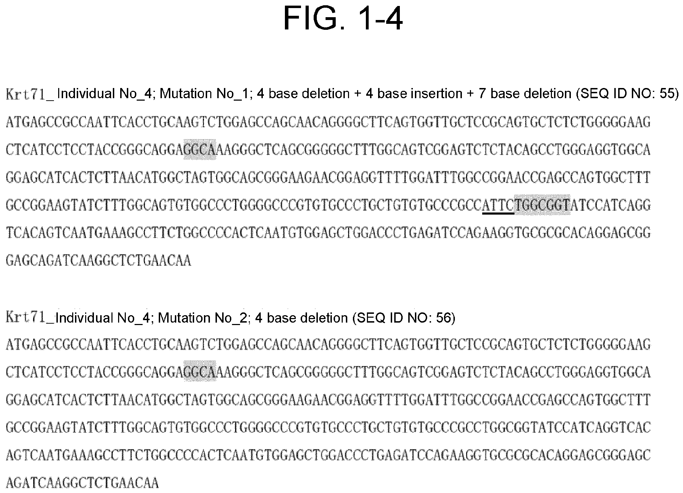

[Figure 1-1] Figure 1-1 shows the sequences of wild-type (WT) Krt71 and Krt71 in each modified individual.
[Figure 1-2] Figure 1-2 shows the sequence of Krt71 in each modified individual.
[Figure 1-3] Figure 1-3 shows the sequence of Krt71 in each modified individual.
[Figure 1-4] Figure 1-4 shows the sequence of Krt71 in each modified individual.
[Figure 2-1] Figure 2-1 shows the sequences of wild-type (WT) Krt72 and Krt72 in each modified individual.
[Figure 2-2] Figure 2-2 shows the sequence of Krt72 in each modified individual.

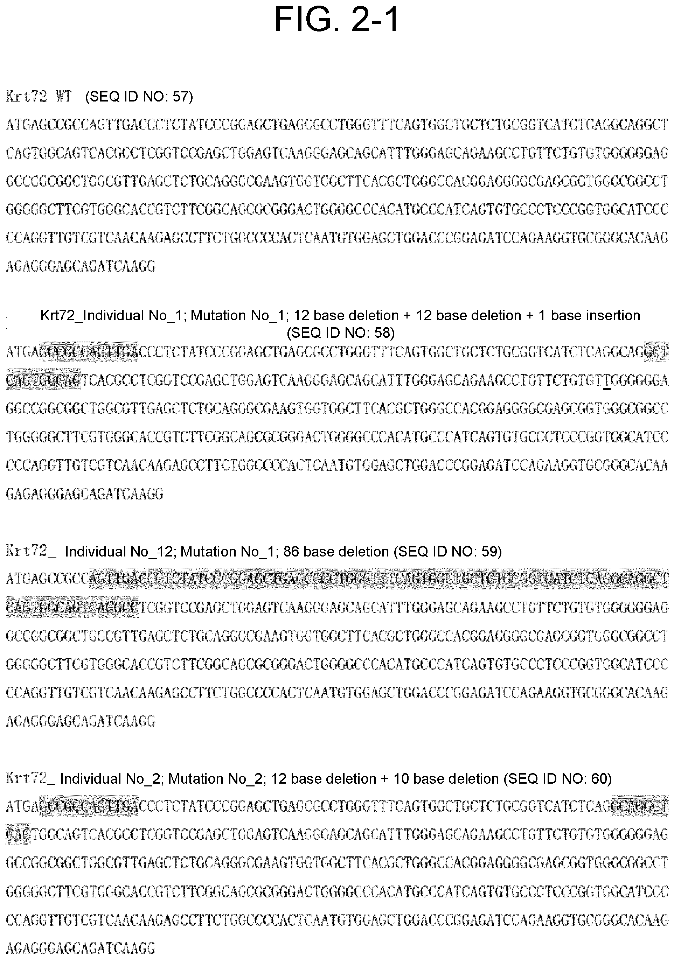

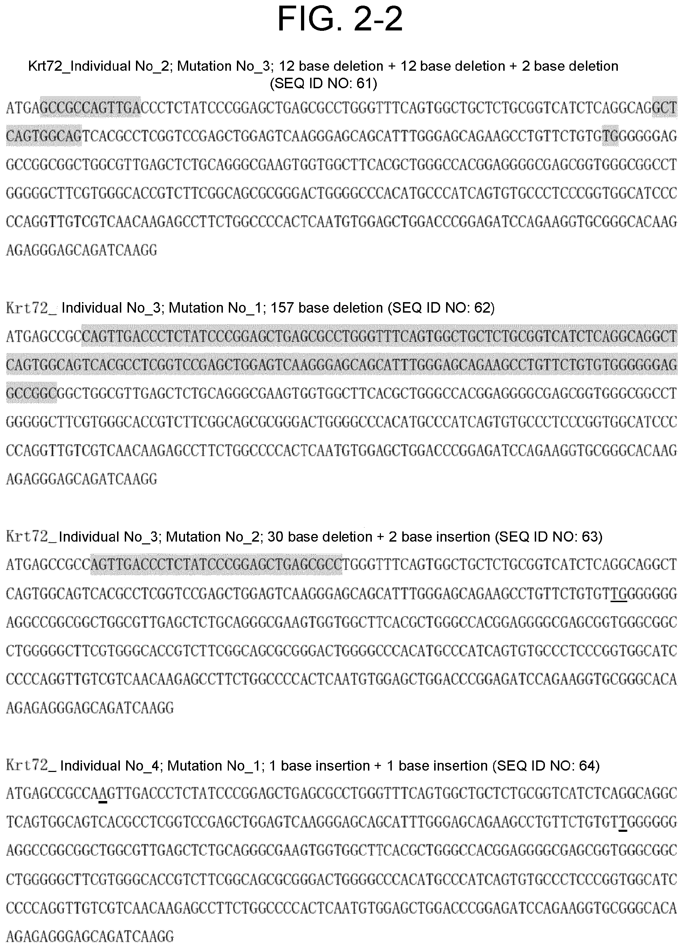

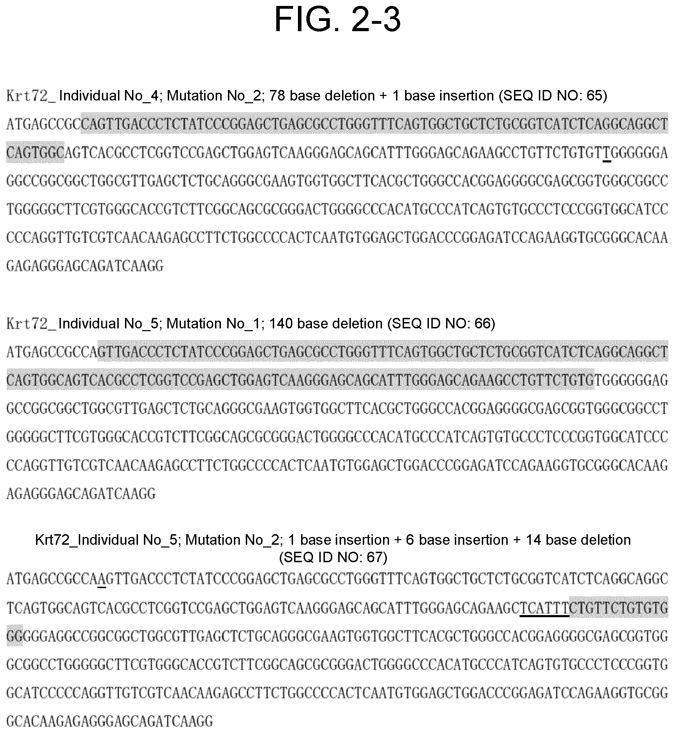

[Figure 2-3] Figure 2-3 shows the sequence of Krt72 in each modified individual.

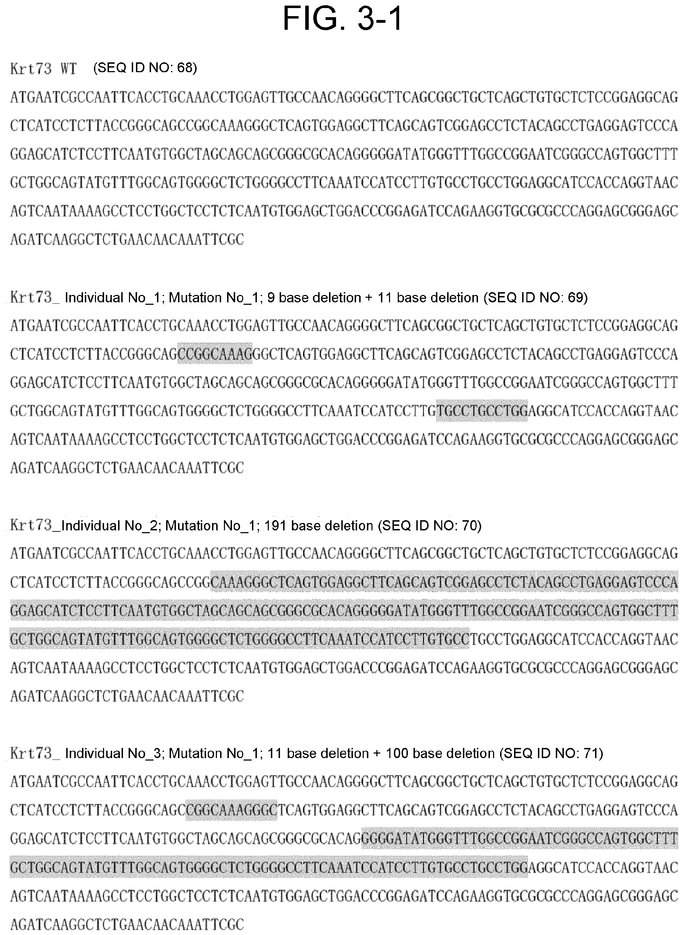

[Figure 3-1] Figure 3-1 shows the sequences of wild-type (WT) Krt73 and Krt73 in each modified individual.

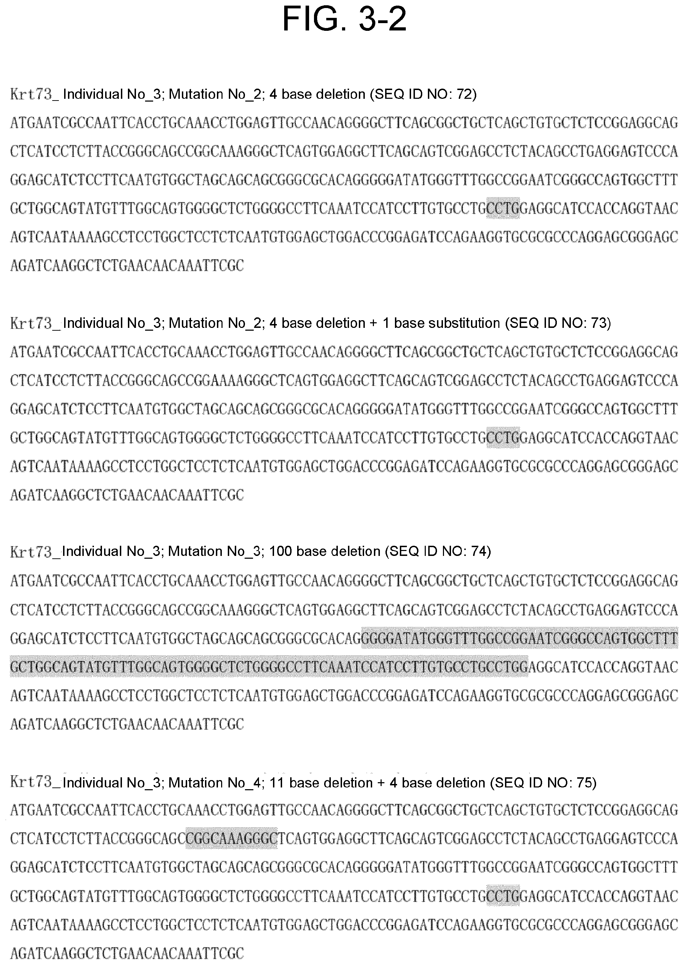

[Figure 3-2] Figure 3-2 shows the sequence of Krt73 in each modified individual.

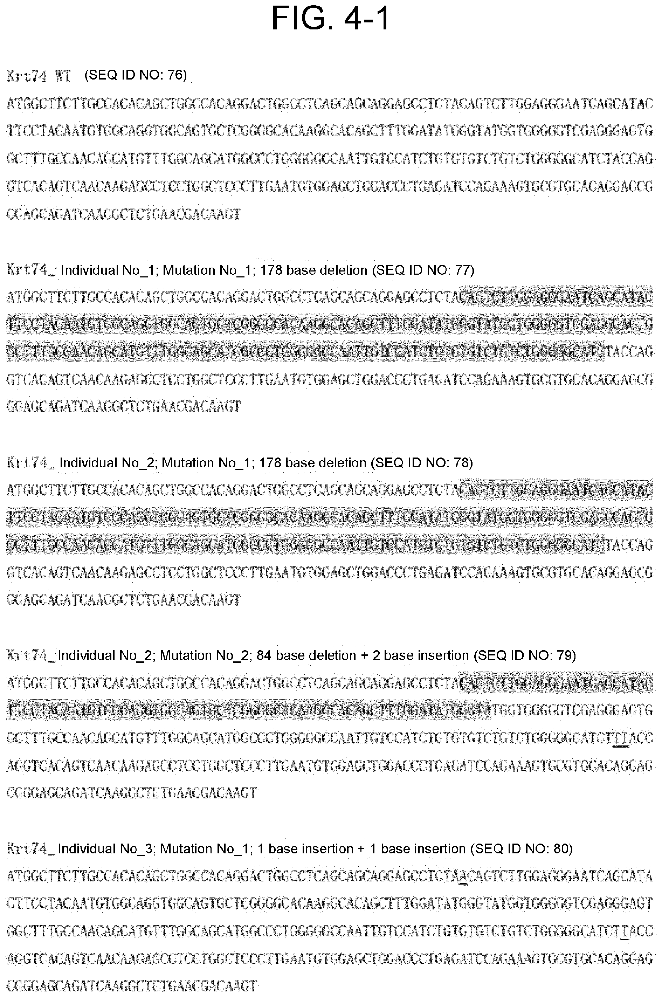

[Figure 4-1] Figure 4-1 shows the sequences of wild-type (WT) Krt74 and Krt74 in each modified individual.

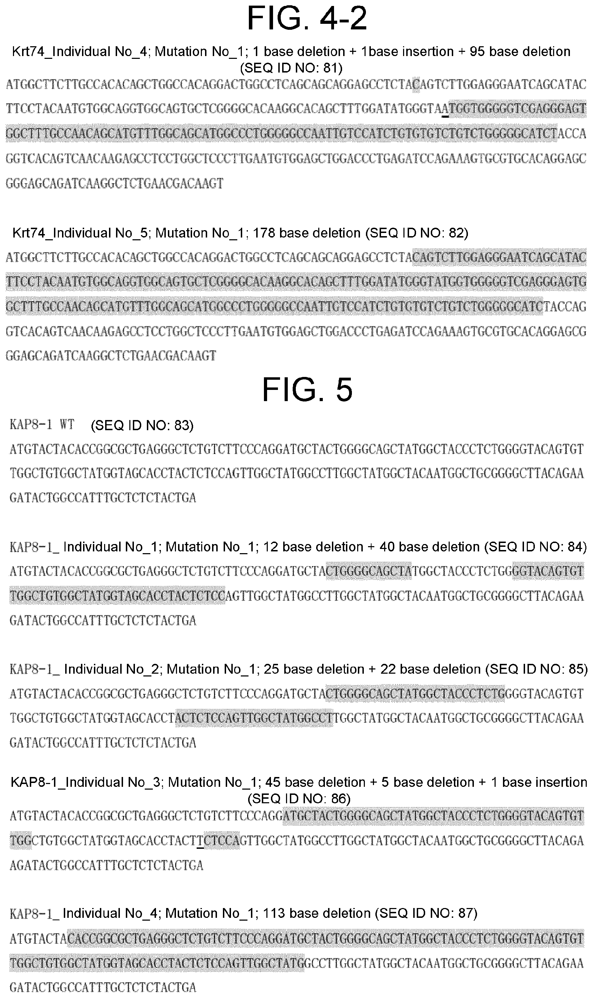

[Figure 4-2] Figure 4-2 shows the sequence of Krt74 in each modified individual.
[Figure 5] Figure 5 shows the sequences of wild-type (WT) KAP8-1 and KAP8-1 in each modified individual.

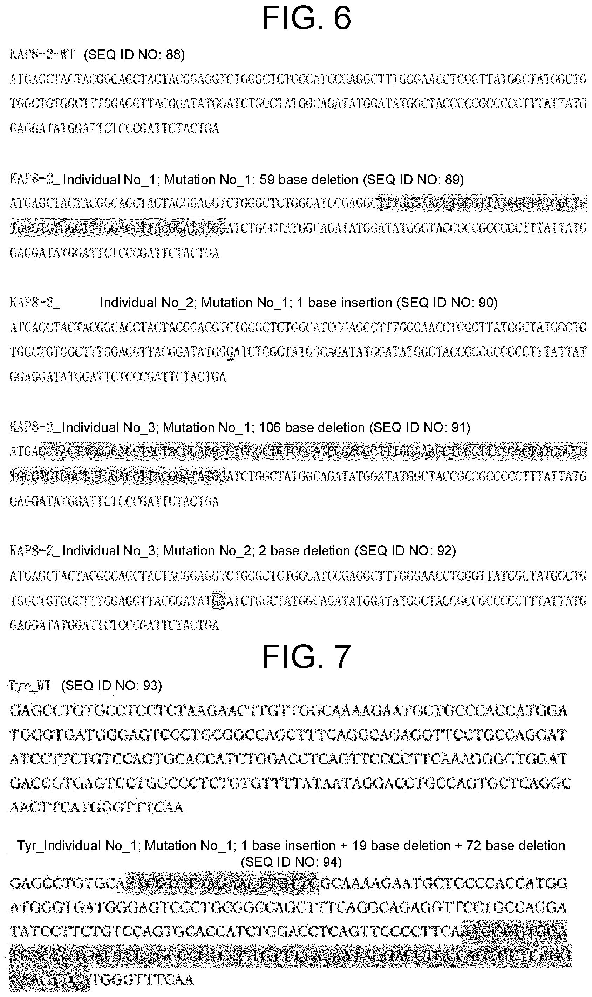

[Figure 6] Figure 6 shows the sequences of wild-type (WT) KAP8-2 and KAP8-2 in each modified individual.
[Figure 7] Figure 7 shows the sequences of wild-type (WT) Tyr and Tyr in each modified individual.

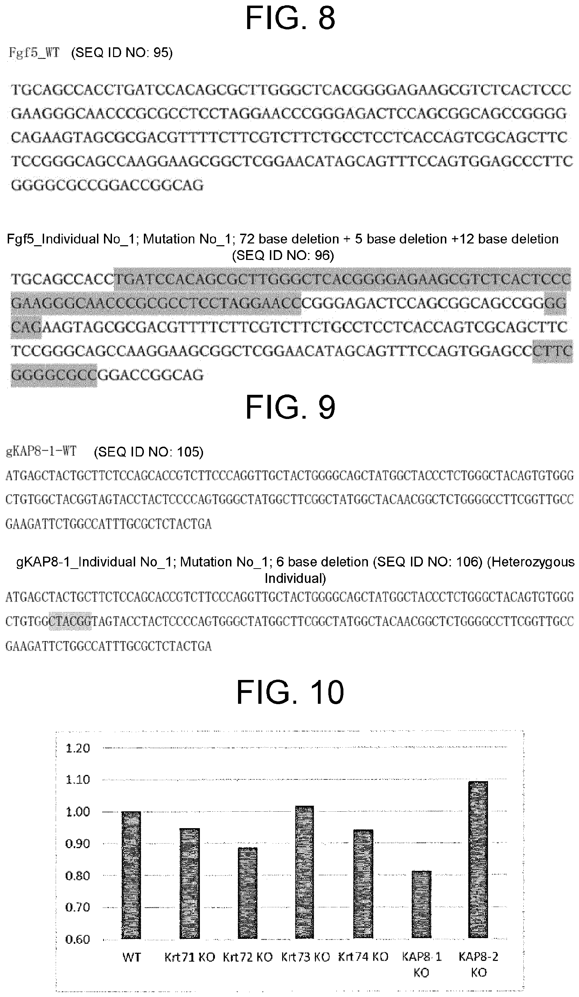

[Figure 8] Figure 8 shows the sequences of wild-type (WT) Fgf5 and Fgf5 in each modified individual.
[Figure 9] Figure 9 shows the sequences of goat wild-type (WT) KAP8-1 and KAP8-1 in each modified individual.
[Figure 10] Figure 10 shows the major axes of animal fibers obtained from a wild-type individual and modified individuals.

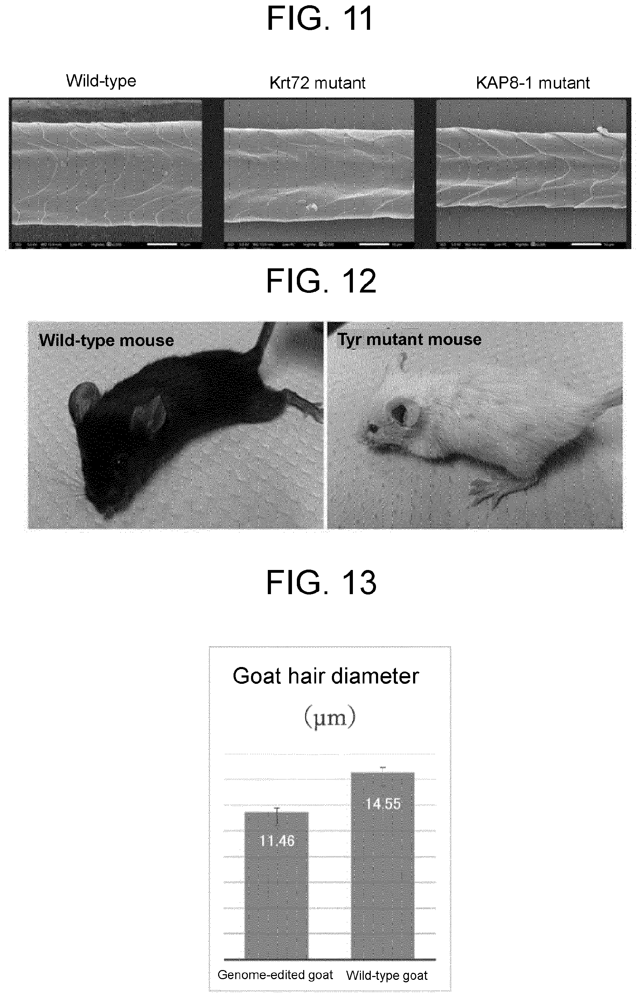

[Figure 11] Figure 11 shows electron micrographs of wild-type hair, hair of a Krt72 mutant, and hair of a KAP8-1 mutant.
[Figure 12] Figure 12 shows the appearance of a wild-type mouse having black hair and a Tyr mutant thereof.
[Figure 13] Figure 13 shows the major axes of hair cross sections of a wild-type goat and a KAP8-1-disrupted goat.

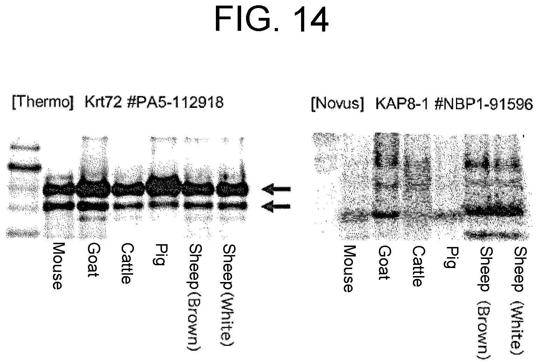

[Figure 14] Figure 14 shows results of Western blotting showing the presence of Krt72 and KAP8-1 proteins contained in the hair of various wild-type animals.

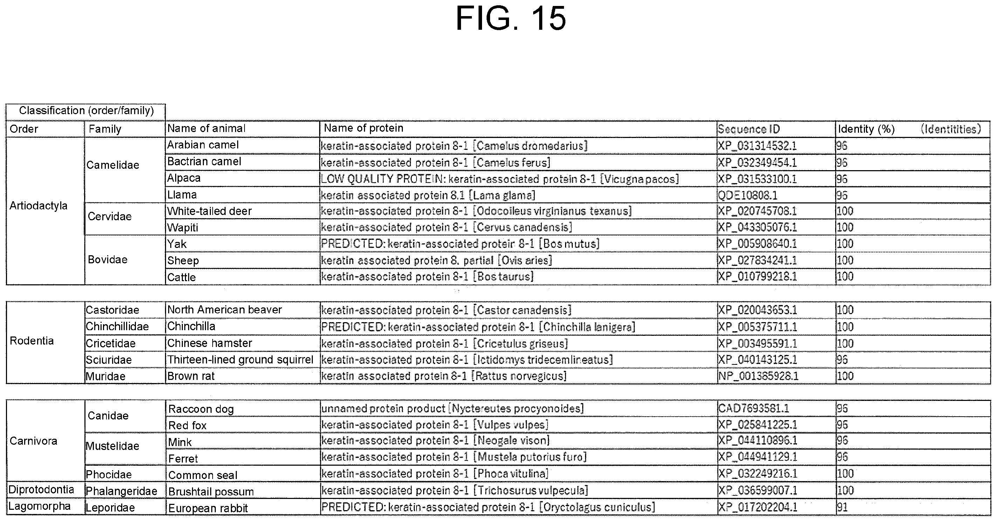

[Figure 15] Figure 15 shows the sequence identity of KAP8-1 among various organism species. Amino acid comparison was performed on the basis of a domain (NCBI-CDD ID: 239162; 23 amino acids) in goat and house mouse KAP8-1 proteins.

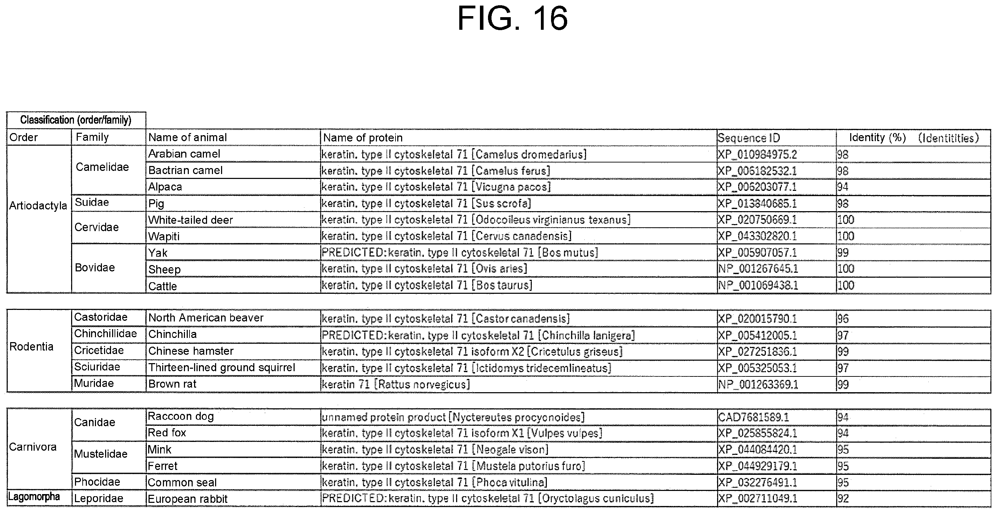

[Figure 16] Figure 16 shows the sequence identity of Krt71 among various organism species. Amino acid comparison was performed on the basis of a domain (NCBI-CDD ID: 365827; 314 amino acids) in goat and house mouse KRT71 proteins. KRT71 proteins of the orders *Artiodactyla* and *Carnivora* were compared with goat KRT71, and KRT71 proteins of the orders *Rodentia* and *Lagomorpha* were compared with house mouse KRT71.

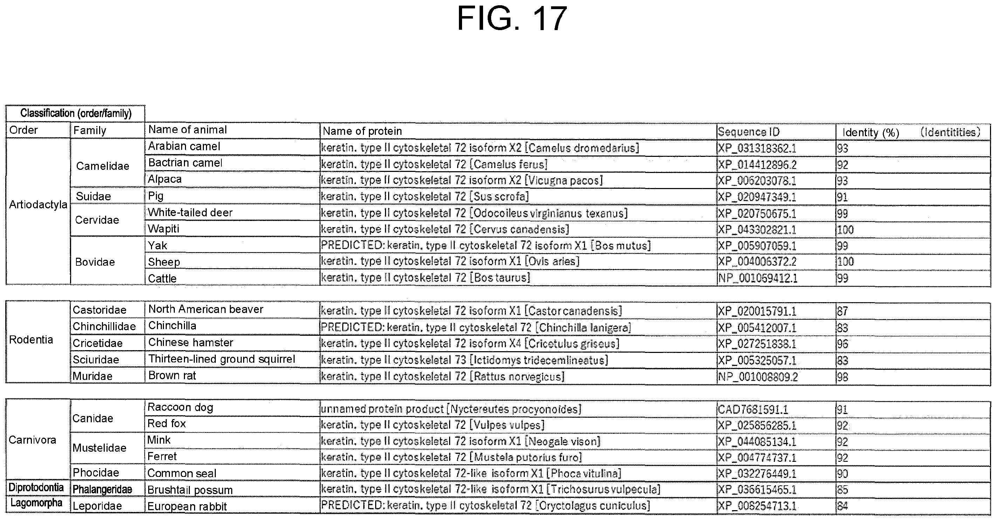

[Figure 17] Figure 17 shows the sequence identity of Krt72 among various organism species. Amino acid comparison was performed on the basis of a domain (NCBI-CDD ID: 365827; 314 amino acids) in goat and house mouse KRT72 proteins. KRT72 proteins of the orders *Artiodactyla, Carnivora,* and *Diprotodontia* were compared with goat KRT72, and KRT72 proteins of the orders *Rodentia* and *Lagomorpha* were compared with house mouse KRT72.

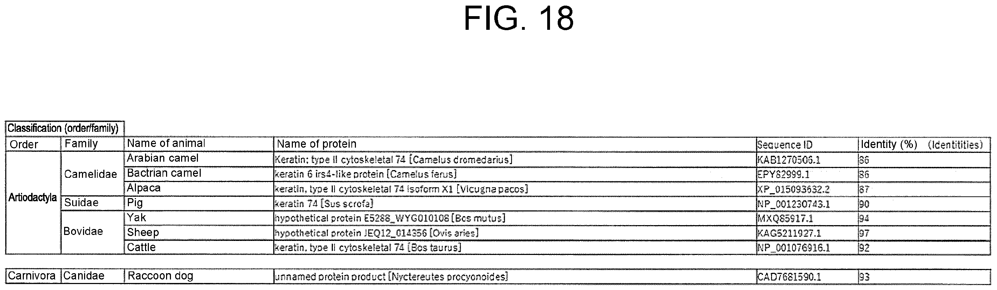

[Figure 18] Figure 18 shows the sequence identity of Krt74 among various organism species. Amino acid comparison was performed on the basis of a domain (NCBI-CDD ID: 365827; 314 amino acids) in goat KRT72 protein.

### Description of Embodiments

All technical terms and scientific terms used herein have the same meanings as those usually understood by those skilled in the art, unless otherwise specified herein. All patents, applications, and other publications (including online information) cited herein are incorporated herein by reference in their entirety.

In the present specification, a term in a singular form does not exclude a plurality. As used herein, the term "comprise" means that an element other than specified matter may be included, and encompasses the term "consist of". The term "consist of" means that an element other than specified matter is not substantially included.

In one aspect, the present invention encompasses a method for preparing a non-human animal, the method comprising reducing expression or functions of at least one gene constituting the Krt or Kap family.

As used herein, the "animal" can be an animal having hair growth of an animal fiber and can be a human or a non-human animal. The non-human animal can be, for example, a non-human mammal or a bird. Examples of the mammal include, but are not limited to, animals of the order *Rodentia* including the families *Muridae, Chinchillidae, Myocastoridae,* and *Catoridae,* the order *Lagomorpha* including the family *Leporidae,* the order *Artiodactyla* (also called cloven-hoofed animal) including the subfamily *Caprinae* and the families *Camelidae* and *Bovidae,* the order *Carnivora* including the families *Canidae, Procyonidae,* and *Mustelidae,* the suborder *Phalangeriformes* including the family *Phalangeridae,* and the order *Didelphimorphia* including the family *Didelphidae.* In one aspect of the present invention, the family *Muridae* encompasses mice (house mice, etc.), rats (roof rats, etc.), and the like. The family *Chinchillidae* encompasses chinchillas and the like. The family *Myocastoridae* encompasses nutrias and the like. The family *Catoridae* encompasses beavers and the like. The family *Leporidae* encompasses domestic rabbits, wild rabbits, Angora rabbits, and the like. The subfamily *Caprinae* encompasses goats, sheep, and the like. The family *Camelidae* encompasses camels (Arabian camels, Bactrian camels, etc.), llamas, vicugnas, alpacas, guanacos, and the like. The family *Bovidae* encompasses yaks, qiviuks (musk-oxen), chirus, and the like. The family *Canidae* encompasses wolfs, raccoon dogs, foxes, and the like. The racoon encompasses raccoons and the like. The family *Mustelidae* encompasses ferrets, minks, sables, and the like. The family *Phocidae* encompasses spotted seals, ringed seals, common seals, ribbon seals, bearded seals, and the like. Examples of the goat include, but are not limited to, cashmere goats, ibexes, Chyangra goats, Angora goats, Shiba goats, Japanese Saanen, alpines, and Tokara goats. Examples of the sheep include, but are not limited to, Merino, Polwarth, Corriedale, Cheviot, Perendale, Romney, Suffolk, Manx Loaghtan, Jacob, and Friesland.

Examples of the bird include, but are not limited to, animals of the order *Anseriformes* including the family *Anatidae,* and the order *Struthioniformes* including the family *Struthionidae.* Examples of the family *Anatidae* include, but are not limited to, mallards, domestic ducks, ducks, swans, wild geese, geese, lesser white-fronted geese, and bean geese. Examples of the *Struthionidae* include, but are not limited to, ostriches.

The non-human animal may be a non-human animal obtained by the crossing of any one of the non-human animals listed above. The non-human animal may be the wild type described above as well as a non-human animal having a modified gene other than the subject gene of the present invention. A mouse, a rabbit, a camel, a goat, or sheep is preferred from the viewpoint of the usefulness of decrease in average fiber diameter by the genetic modification of the present invention. The goat is particularly preferably a cashmere goat.

The animal fiber may be hair of the human head.

The form of the non-human animal may be an embryo form or an individual form. The embryo refers to an arbitrary cell or cell population having ontogenetic ability. Examples of the embryo include fertilized eggs, early embryos, morulae, blastocysts, and inner cell masses contained therein as well as induced pluripotent stem (iPS) cells and embryonic stem (ES) cells. In the individual, an animal fiber develops (hair growth) from body surface by raising. The embryo can be transplanted into the uterus of a non-human animal and thereby allowed to grow into an individual. The embryo or the individual may be a mosaic or a chimera. When reduction in expression or functions of a gene is caused by genetic modification of the subject gene, even a mosaic or chimeric embryo or individual can contain modification at least in a cell constituting the hair follicle or a germline cell. An animal fiber having a thin fiber diameter can develop when a cell constituting the hair follicle has the modification. Progeny in which an animal fiber having a thin fiber diameter develops can be obtained when a germline cell has the modification. The crossing of homozygous genetically modified animals theoretically produces a homozygous genetically modified animal with 100% probability. The crossing of a heterozygous genetically modified animal and a homozygous genetically modified animal theoretically produces a homozygous genetically modified animal with 50% probability. The crossing of heterozygous genetically modified animals theoretically produces a homozygous genetically modified animal with 25% probability. The genetic modification is preferably homozygous genetic modification, i.e., is performed at chromosomes from both parents.

Examples of the cell constituting the hair follicle include, but are not limited to, hair matrix cells (epithelial cells) contained in the hair matrix, pigment-producing cells (melanocytes), cuboidal epithelial cells contained in the root sheath, hair follicle stem cells and pigment stem cells contained in the hair bulge, follicle dermal papilla cells, and dermal sheath cup cells. The hair follicle may be single follicle or may be compound follicle. The single follicle may be primary hair follicle or may be secondary hair follicle. The germline refers to, for example, but not limited to, gamete such as ovum or sperm, or a primordial germ cell, an oogonium, an oocyte, an ooblast, a spermatogonia, or a sperm cell serving as a basis thereof.

As used herein, the "animal fiber" refers to an animal fiber of the human or the non-human animal mentioned above. The animal fiber refers to, for example, a fur fiber when the non-human animal is a mammal, and a feather fiber when the non-human animal is a bird. As used herein, the "animal fiber" is also referred to as "hair" or "body hair". The fur fiber is typically an animal fiber derived from the mammal described above in detail. Specific examples thereof include mouse hair, rat hair, chinchilla hair "chinchilla", domestic rabbit or wild rabbit hair "rabbit", Angora rabbit hair "Angora", cashmere goat hair "cashmere", Angora goat hair "mohair", sheep hair "sheep wool" or "wool", alpaca hair "alpaca", llama hair "llama", Bactrian camel hair "camel", yak hair "yak", ibex hair "ibex", guanaco hair "guanaco", qiviuk hair "qiviuk", chiru hair "chiru", raccoon dog hair, raccoon hair "raccoon", mink hair "mink", sable hair "sable", possum hair "possum", fox hair "fox", opossum hair "opossum", beaver hair "beaver", seal hair "seal", nutria hair "nutria", ferret hair "ferret", and vicugna hair "vicuna". As is evident from these examples, in the present invention, the same name may be used for the name of an animal and the name of its fur. In the present invention, the fur fiber may be guard hair or may be underlayer (also called underhair, undercoat, underfur, or vellus hair). The cashmere is body hair mainly containing underlayer or consisting of underlayer, which is obtained by removing guard hair from body hair obtained from a cashmere goat.

The feather fiber is not limited as long as the fiber is derived from a bird. The feather fiber is typically an animal fiber derived from the bird described above in detail. Specific examples thereof include wild duck, goose, duck, quail, chicken, turkey, long-tailed cock, bantam, dove, ostrich, pheasant, guinea fowl, or ostrich feather. The feather fiber includes, but not limited to, contour feather, plume, semi-plume, and the like. The animal fiber of the present invention encompasses raw hair cut from the non-human animal as well as raw hair subjected to selection, washing, or cutting, etc., and/or such hair subjected to arbitrary processing, for example, scale peeling treatment, enzymatic treatment, or pulse treatment.

As used herein, the "Krt family" refers to a gene family encoding keratin protein. As used herein, the "at least one gene constituting the Krt family" is also referred to as Krt gene. A protein encoded by the Krt gene is also referred to as Krt protein. In general, the Krt gene or the Krt protein is also represented by a symbol, for example, but not limited to, Ck, CK, KRT, or K in addition to Krt. In the present specification, genes or proteins represented by these symbols are represented by the symbol "Krt".

In the present invention, the Krt gene can be a member of the Krt gene retained in the genome of a non-human animal and can be preferably a gene encoding type II (neutral to basic) keratin protein. In the present invention, the Krt gene is preferably at least one gene selected from the group consisting of Krt71, Krt72, Krt73, and Krt74, more preferably at least one gene selected from the group consisting of Krt71, Krt72, and Krt74, further preferably Krt72.

In one aspect of the present invention, the Krt gene can be Krt gene expressed in a cell constituting the hair follicle while a non-human animal individual grows. The phrase "while an individual grows" refers to, particularly, a period when an animal fiber develops (hair grows) from the non-human animal. The Krt gene can be expressed only in a particular period such as a particular time or season. Particularly, Krt gene expressed in a period with a high amount of hair grown is preferred. The hair follicle is not particularly limited as long as the hair follicle constitutes a non-human animal.

Such expression can be measured by a method such as RT-PCR, qRT-PCR, Northen blot, expression arrays, or RNA sequencing usually used in the transcriptome analysis of the hair follicle. Those skilled in the art may perform such measurement with reference to a method used in Qiao et al., Genet Mol Res. 2016 Sep 2; 15(3), Din et al., BMC Genomics. 2019 Feb 15; 20(1): 140, Fan et al., Genet Mol Res. 2015 Dec 22; 14(4): 17904-15, Zhu PLoS One. 2013 Sep 19; 8(9): e76282 or the like or with reference to a gene described in these literatures.

Such expression in the hair follicle is reflected as a constituent protein in an animal fiber. Therefore, a protein expressed in the hair follicle can be predicted by conducting the proteome analysis of the animal fiber instead of the direct transcriptome analysis of the hair follicle. An approach usually used can be adopted as the proteome analysis of the animal fiber and can involve, for example, dissolving a protein in the animal fiber, and conducting analysis by a method such as electrophoresis, mass spectrometry, or immunoassay. Those skilled in the art can conduct such analysis with reference to Plowman et al., Electrophoresis. 2000 May; 21(9): 1899-906, Plowman et al., J Proteomics. 2012 Jul 19; 75(14): 4315-24, Li et al., PLoS One. 2016 Jan 20; 11(1): e0147044 or the like.

The animal fiber is composed of a hair shaft portion which resides outside the epidermis and a hair root portion which resides in the hair follicle. As for the structure of the animal fiber in the hair follicle, the hair shaft composed of the cuticle, the hair cortex, and the hair medulla is located at the center of the hair follicle and surrounded on its outer side by the inner root sheath and on its outermost side by the external root sheath via the companion layer. In the growth of the animal fiber, the proliferation, differentiation, and keratinization of hair matrix cells present in a hair bulb portion (hair matrix) located beneath the hair root portion partially occupy a central process.

Specifically, hair matrix cells that have proliferated in the hair root portion differentiate into cells constituting the inner root sheath and three types of hair shaft cells constituting the cuticle, the hair cortex, and the hair medulla, and proliferate and keratinize while specifically expressed at each site.

For such a growth process of the animal fiber, Krt71, Krt72, Krt73, and Krt74 proteins are known as Krt protein expressed in the inner root sheath (see Jeffrey E Plowman; Duane P Harland, Singapore Springer Singapore 2018, Hair Fibre: Proteins, Structure and Development). At least one gene selected from the group consisting of Krt71, Krt72, Krt73, and Krt74 is modified in a tissue containing at least the inner root sheath. This modification may be specific for the tissue containing the inner root sheath.

The present inventors have found that the fiber diameter of an animal fiber can be decreased or smooth fiber surface can be imparted thereto, by reducing expression or functions of at least one gene constituting the non-human animal Krt family, in particular, a gene encoding Krt protein expressed in the inner root sheath, cuticle, hair cortex, or hair medulla of the non-human animal. The obtained animal fiber maintained a healthy layer structure and toughness, etc.

Thus, in one aspect, the present invention includes a method for preparing a non-human animal, comprising reducing expression or functions of at least one gene encoding Krt protein expressed in a tissue containing the inner root sheath, cuticle, hair cortex, or hair medulla of a non-human animal.

In another aspect of the present invention, the at least one gene constituting the Krt family can be a gene expressed in the inner root sheath, i.e., a gene encoding at least one member selected from the group consisting of Krt71, Krt72, Krt73, and Krt74.

The phylogenetic analysis of keratin including subfamily classification is known in the art and described in Eckhart and Ehrlich, Adv Exp Med Biol. 2018; 1054: 33-45.

Genes encoding proteins constituting each of these subfamilies are found to have sequence homology in addition to a common timing of expression and expression site and therefore considered to have the same functions. Thus, the same character can be obtained on a subfamily basis by reducing expression or functions of genes encoding proteins within each subfamily.

In one aspect, the Krt gene, expression or functions of which are to be reduced according to the present invention is preferably at least one gene selected from the group consisting of Krt71, Krt72, Krt73, and Krt74, more preferably Krt71, Krt72, or Krt74, particularly preferably Krt72. In the case of modifying at least one gene selected from the group consisting of Krt71, Krt72, Krt73, and Krt74 (particularly, Krt71), the modified animal can be
an animal of the order *Rodentia* including the family *Muridae* (except for a mouse), *Chinchillidae, Myocastoridae, Catoridae,* or the like; the order *Lagomorpha* including the family *Leporidae* or the like; the order *Artiodactyla* (also called cloven-hoofed animal; except for a bovine) including the subfamily *Caprinae,* the family *Camelidae* or *Bovidae,* or the like; the order *Carnivora* including the family *Canidae, Procyonidae, Mustelidae,* or the like; the suborder *Phalangeriformes* including the family *Phalangerida* or the like; or the order *Didelphimorphia* including the family *Didelphidae* or the like, and can be
an animal of the family *Chinchillidae, Myocastoridae,* or *Catoridae*; the order *Lagomorpha* including the family *Leporidae* or the like; the subfamily *Caprinae* or the family *Camelidae*; the order *Carnivora* including the family *Canidae, Procyonidae, Mustelidae,* or the like; the suborder *Phalangeriformes* including the family *Phalangerida* or the like; or the order *Didelphimorphia* including the family *Didelphidae* or the like. Thus, the animal fiber can also be an animal fiber of any of these modified animals. In one aspect, the gene to be modified comprises no Krt71.

In one aspect of the present invention, the Krt gene may be a gene encoding a minor component among protein components constituting the animal fiber of a human or a non-human animal. The animal fiber has approximately 50 to 60% of keratin and approximately 20 to 30% of matrix protein including keratin associated protein (KAP). The gene encoding a minor component is a gene encoding Krt protein ranked, for example, in the top 10 or lower, 20 or lower, or 30 or lower when the animal fiber is subjected to mass spectrometry, among the Krt and KAP proteins. Typically, for example, Krt86, Krt87, Krt83, Krt85, Krt84, Krt33a, Krt31, Krt34, Krt33b, Krt35, and Krt32 are genes encoding major components, and genes encoding components other than these major components are genes encoding minor components. The Krt gene is, for example, a gene encoding Krt71, Krt72, or Krt74.

As used herein, the "KAP family" refers to a gene family encoding keratin associated protein. As used herein, the "at least one gene constituting the KAP family" is also referred to as KAP gene. A protein encoded by the KAP gene is also referred to as KAP protein. In general, the KAP gene or the KAP protein is also represented by a symbol, for example, Kap, Krtap, or KRTAP in addition to KAP. In the present specification, genes or proteins represented by these symbols are represented by the symbol "KAP".

The KAP gene can be a gene encoding, for example, high glycine-tyrosine KAP protein as long as the gene is a member of the KAP family retained in the genome of the non-human animal of the present invention.

In one aspect of the present invention, examples of the gene encoding high glycine-tyrosine KAP protein include genes of the KAP8 subfamilies. Examples of the KAP8 subfamilies include KAP8-1 and KAP8-2.

In one aspect of the present invention, the KAP gene is preferably KAP gene encoding high glycine-tyrosine KAP protein. The KAP encoding high glycine-tyrosine KAP protein is preferably a member of the KAP8 subfamilies. KAP8 is particularly preferably KAP8-1. Examples of the modification of KAP8-1 include, but are not particularly limited to, frameshift mutation, nonsense mutation, missense mutation, deletion, and insertion. In a preferable aspect, the frameshift mutation and the nonsense mutation can be allowed to occur at, for example, exon 1 or exon 2 (preferably exon 1) on the 5'-terminal side of the gene to be disrupted. The deletion can be, for example, deletion of 1 or more amino acids long or 2 or more amino acids long, preferably 3 or more amino acids long, more preferably 4 or more amino acids long, 5 or more amino acids long, 6 or more amino acids long, 7 or more amino acids long, 8 or more amino acids long, 9 or more amino acids long, 10 or more amino acids long, 20 or more amino acids long, 30 or more amino acids long, 40 or more amino acids long, 50 or more amino acids long, or 60 or more amino acids long. The deletion can also occur in, for example, an amino acid of KAP8-1 corresponding to any one or more amino acids at position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 of an amino acid sequence registered under GenBank: AAS00529.1. The deletion is capable of inducing reduction in or loss of functions of KAP8-1 protein. Examples of the frameshift mutation include insertion or deletion of n bases long {wherein n is a natural number that is not a multiple of 3} in an exon region of a nucleic acid encoding KAP8-1. n can be, for example, a natural number of 1 or larger, 2 or larger, 4 or larger, 5 or larger, 7 or larger, 8 or larger, 10 or larger, 11 or larger, 13 or larger, 14 or larger, 16 or larger, 17 or larger, 19 or larger, 20 or larger, 30 or larger, 40 or larger, 50 or larger, 60 or larger, 70 or larger, 80 or larger, 90 or larger, 100 or larger, 110 or larger, 120 or larger, 130 or larger, 140 or larger, or 150 or larger. The frameshift is capable of inducing reduction in or loss of functions of KAP8-1 protein. The nonsense mutation can occur in, for example, a region of KAP8-1 corresponding to any one or more amino acids at position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 of an amino acid sequence registered under GenBank: AAS00529.1. The missense mutation can occur in, for example, a region of KAP8-1 corresponding to any one or more amino acids at position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 of an amino acid sequence registered under GenBank: AAS00529.1. However, the nonsense mutation and the missense mutation are capable of inducing reduction in or loss of functions of KAP8-1. The deletion mutation of KAP8-1 can be, for example, deletion of amino acids of KAP8-1 corresponding to positions 30 and 31 of an amino acid sequence registered under GenBank: AAS00529.1. The amino acid corresponding to a particular position of an amino acid sequence is an amino acid that is aligned to the same position when two or more amino acid sequences are aligned. For example, the amino acids of KAP8-1 corresponding to positions 30 and 31 of an amino acid sequence registered under GenBank: AAS00529.1 means amino acids of KAP8-1 that are aligned to the same positions as those of the amino acids at positions 30 and 31 when the amino acid sequence registered under GenBank: AAS00529.1 and the amino acid sequence of KAP8-1 are aligned.

In one aspect of the present invention, the genetically modified animal has reduction in expression or functions of at least one gene selected from the group consisting of Krt71, Krt72, and Krt74, and KAP8-1 gene, or disruption of the genes. In one aspect of the present invention, the genetically modified animal has reduction in expression or functions of Krt71 and KAP8-1, or disruption of the genes. The genetically modified animal has reduction in expression or functions of Krt74 and KAP8-1, or disruption of the genes.

In a preferable aspect of the present invention, the genetically modified animal has reduction in expression or functions of Krt72 and KAP8-1, or disruption of the genes.

In a further preferable aspect of the present invention, the genetically modified animal has reduction in expression or functions of
Krt71, Krt72, and KAP8-1;
Krt72, Krt74, and KAP8-1; or
Krt72, Krt74, and KAP8-1,
or disruption of the genes.

In a further preferable aspect of the present invention, the genetically modified animal has reduction in expression or functions of Krt71, Krt72, Krt74, and KAP8-1, or disruption of the genes.

In one aspect of the present invention, the genetically modified animal is a male or a female. If a male animal fiber or a female animal fiber on an animal fiber basis depends on required characteristics of the animal fiber, a male or a female can be selected according to the required characteristics of the animal fiber to obtain an animal fiber thereof. In one aspect of the present invention, the genetically modified animal can have a given age.

In one aspect of the present invention, the genetically modified animal can be selected as a genetically modified animal having an average fiber diameter (major axis) of an animal fiber (particularly, body hair) that is 95% or less, 90% or less, 85% or less, or 80% or less of the average fiber diameter (major axis) of the animal fiber (particularly, body hair) of the same animal having no modification of the gene. This provides a genetically modified animal having modification of a gene, wherein an average fiber diameter (major axis) of an animal fiber (particularly, body hair) thereof is 95% or less, 90% or less, 85% or less, or 80% or less of the average fiber diameter (major axis) of the animal fiber (particularly, body hair) of a syngeneic or allogeneic animal having no modification of the gene, and the modification of the gene has reduction in expression or functions of at least one gene selected from the group consisting of Krt71, Krt72, Krt74, and KAP8-1, or disruption of the gene. The control animal is preferably syngeneic. If such a syngeneic control animal cannot be obtained, an allogeneic animal can be used. The allogeneic animal is preferably an allogeneic wild-type animal, more preferably an animal before modification. An effect of genetic modification can be most properly evaluated by comparison between before and after modification.

In one aspect, the present invention encompasses the method comprising reducing expression or functions of at least one gene selected from one or more genes constituting the Krt or KAP family.

In one aspect, the present invention includes reducing expression or functions of at least one gene selected from the group consisting of Krt71, Krt72, and Krt74, and KAP8-1 gene, or disrupting (knocking out) the genes.

As used herein, the Fgf5 gene refers to a gene encoding FGF5 (fibroblast growth factor 5). In general, the gene is also represented by a symbol, for example, but not limited to, FGF-5 or HBGF-5 in addition to Fgf5. In the present specification, genes represented by these symbols are represented by "Fgf5".

As used herein, the Tyr gene refers to a gene encoding tyrosinase. In general, the gene is also represented by a symbol, for example, but not limited to, TYR, LB24-AB, or SK29-AB in addition to Tyr. In the present specification, genes represented by these symbols are represented by "Tyr".

In the present specification, the Krt gene, the Kap gene, the Fgf5 gene, and the Tyr gene are also collectively referred to as a "subject gene". A protein encoded by the subject gene is also referred to as a "subject protein".

As used herein, the "homologs" refer to a group of highly similar genes having the same evolutionary origin. The homologs are classified into two types, "orthologs" and "paralogs". The orthologs refer to homologs derived from the same gene upon speciation. The paralogs refer to homologs arising through gene duplication within the same species.

Krt71 has a nucleic acid sequence and an amino acid sequence known in various organism species. Mouse Krt71 has, for example, an amino acid sequence registered under GenBank: AAI25349.1 or an amino acid sequence of Krt71 corresponding to the amino acid sequence. Goat Krt71 has, for example, an amino acid sequence registered under NCBI Reference Sequence: XP_017903206.1 or an amino acid sequence of Krt71 corresponding to the amino acid sequence. Sheep Krt71 has, for example, an amino acid sequence registered under GenBank: AFV46425.1 or an amino acid sequence of Krt71 corresponding to the amino acid sequence. Rabbit Krt71 has, for example, an amino acid sequence registered under NCBI Reference Sequence: XP_002711049.1 or an amino acid sequence of Krt71 corresponding to the amino acid sequence. Mink Krt71 has, for example, an amino acid sequence registered under NCBI Reference Sequence: XP_044084420.1 or an amino acid sequence of Krt71 corresponding to the amino acid sequence. Human Krt71 has, for example, an amino acid sequence registered under GenBank: AAI03919.1 or an amino acid sequence of Krt71 corresponding to the amino acid sequence. Those skilled in the art should be able to determine the Krt71 gene and the amino acid sequence of the protein in each organism species.

Krt72 has a nucleic acid sequence and an amino acid sequence known in various organism species. Mouse Krt72 has, for example, an amino acid sequence registered under GenBank: EDL04025.1 or an amino acid sequence of Krt72 corresponding to the amino acid sequence. Goat Krt72 has, for example, an amino acid sequence registered under NCBI Reference Sequence: XP_013832621.2 or an amino acid sequence of Krt72 corresponding to the amino acid sequence. Sheep Krt72 has, for example, an amino acid sequence registered under NCBI Reference Sequence: XP_004006372.2 or NCBI Reference Sequence: XP_042102628.1 or an amino acid sequence of Krt72 corresponding to the amino acid sequence. Rabbit Krt72 has, for example, an amino acid sequence registered under NCBI Reference Sequence: XP_008254713.1 or an amino acid sequence of Krt72 corresponding to the amino acid sequence. Mink Krt72 has, for example, an amino acid sequence registered under NCBI Reference Sequence: XP_044085134.1 or NCBI Reference Sequence: XP_044085135.1 or an amino acid sequence of Krt72 corresponding to the amino acid sequence. Human Krt72 has, for example, an amino acid sequence registered under GenBank: AAI13687.1 or an amino acid sequence of Krt72 corresponding to the amino acid sequence. Those skilled in the art should be able to determine the Krt72 gene and the amino acid sequence of the protein in each organism species.

Krt74 has a nucleic acid sequence and an amino acid sequence known in various organism species. Mouse Krt74 has, for example, an amino acid sequence registered under GenBank: EDL04026.1 or an amino acid sequence of Krt74 corresponding to the amino acid sequence. Goat Krt74 has, for example, an amino acid sequence registered under NCBI Reference Sequence: XP_005701908.2 or an amino acid sequence of Krt74 corresponding to the amino acid sequence. Sheep Krt74 has, for example, an amino acid sequence registered under NCBI Reference Sequence: XP_004006373.1 or an amino acid sequence of Krt74 corresponding to the amino acid sequence. Mink Krt74 has, for example, an amino acid sequence registered under NCBI Reference Sequence: XP_044085217.1 or an amino acid sequence of Krt74 corresponding to the amino acid sequence. Human Krt74 has, for example, an amino acid sequence registered under GenBank: KAI2565848.1 or an amino acid sequence of Krt74 corresponding to the amino acid sequence. Those skilled in the art should be able to determine the Krt74 gene and the amino acid sequence of the protein in each organism species.

Keratin associated protein 8-1 (KAP8-1) has a nucleic acid sequence and an amino acid sequence known in various organism species. Mouse KAP8-1 has, for example, an amino acid sequence registered under NCBI Reference Sequence: NP_034805.1 or an amino acid sequence of KAP8-1 corresponding to the amino acid sequence. Goat KAP8-1 has, for example, an amino acid sequence registered under GenBank: AAS00529.1 or an amino acid sequence of KAP8-1 corresponding to the amino acid sequence. Sheep KAP8-1 has, for example, an amino acid sequence registered under NCBI Reference Sequence: XP_027834241.1 or an amino acid sequence of KAP8-1 corresponding to the amino acid sequence. Rabbit KAP8-1 has, for example, an amino acid sequence registered under NCBI Reference Sequence: XP_017202204.1 or an amino acid sequence of KAP8-1 corresponding to the amino acid sequence. Mink KAP8-1 has, for example, an amino acid sequence registered under NCBI Reference Sequence: XP_044110896.1 or an amino acid sequence of KAP8-1 corresponding to the amino acid sequence. Human KAP8-1 has, for example, an amino acid sequence registered under NCBI Reference Sequence: NP_787053.1 or an amino acid sequence of KAP8-1 corresponding to the amino acid sequence. Those skilled in the art should be able to determine the KAP8-1 gene and the amino acid sequence of the protein in each organism species.

Fibroblast growth factor 5 (Fgf5) has a nucleic acid sequence and an amino acid sequence known in various organism species. Mouse Fgf5 has, for example, an amino acid sequence registered under GenBank: AAH71227.1 or an amino acid sequence of Fgf5 corresponding to the amino acid sequence. Goat Fgf5 has, for example, an amino acid sequence registered under GenBank: AIZ78004.1 or an amino acid sequence of Fgf5 corresponding to the amino acid sequence. Sheep Fgf5 has, for example, an amino acid sequence registered under GenBank: AID52934.1 or an amino acid sequence of Fgf5 corresponding to the amino acid sequence. Rabbit Fgf5 has, for example, an amino acid sequence registered under NCBI Reference Sequence: XP_008265908.1 or an amino acid sequence of Fgf5 corresponding to the amino acid sequence. Mink Fgf5 has, for example, an amino acid sequence registered under NCBI Reference Sequence: XP_044080065.1 or an amino acid sequence of Fgf5 corresponding to the amino acid sequence. Human Fgf5 has, for example, an amino acid sequence registered under GenBank: AAB06463.1 or an amino acid sequence of Fgf5 corresponding to the amino acid sequence. Those skilled in the art should be able to determine the Fgf5 gene and the amino acid sequence of the protein in each organism species. Fgf5 is expressed in the external root sheath and can thus be modified in a tissue containing the external root sheath. The modification may be specific for the tissue.

Tyrosinase (tyr) has a nucleic acid sequence and an amino acid sequence known in various organism species. Mouse Tyr has, for example, an amino acid sequence registered under GenBank: BAA00341.1 or an amino acid sequence of tyr corresponding to the amino acid sequence. Goat Tyr has, for example, an amino acid sequence registered under GenBank: AEV91332.1 or an amino acid sequence of tyr corresponding to the amino acid sequence. Sheep tyr has, for example, an amino acid sequence registered under GenBank: ACF21682.1 or an amino acid sequence of tyr corresponding to the amino acid sequence. Rabbit Tyr has, for example, an amino acid sequence registered under NCBI Reference Sequence: NP_001075546.1 or an amino acid sequence of tyr corresponding to the amino acid sequence. Mink tyr has, for example, an amino acid sequence registered under GenBank: AJO15925.1 or an amino acid sequence of tyr corresponding to the amino acid sequence. Human tyr has, for example, an amino acid sequence registered under GenBank: AAB60319.1 or an amino acid sequence of Tyr corresponding to the amino acid sequence.

Orthologs or homologs of a protein having any of the amino acid sequences described above have high homology to any of these amino acid sequences. The high homology means 50% or more or 60% or more, preferably 70% or more, further preferably 80% or more, more preferably 90% or more (e.g., 95% or more and further 96%, 97%, 98%, or 99% or more) homology. This homology can be determined with default parameters of the protein BLAST algorithm provided by National Center for Biotechnology Information (NCBI).

A nucleic acid sequence encoding any of the amino acid sequences mentioned above can be determined by nucleic acid sequence search in NCBI National Library of Medicine. The nucleic acid sequence may be determined from a codon usage table on the basis of the amino acid sequence. The nucleic acid sequence may be codon-optimized for a cell expressing the nucleic acid sequence. DNA having a nucleotide sequence encoding any of the proteins described above can be isolated by exploiting its hybridization under stringent conditions to DNA having a natural nucleic acid sequence. In this context, the "stringent conditions" are "2 × SSC, 0.1% SDS, and 50°C", "2 × SSC, 0.1% SDS, and 42°C", or "1 × SSC, 0.1% SDS, and 37°C". Examples of more stringent conditions can include conditions such as "2 × SSC, 0.1% SDS, and 65°C", "0.5 × SSC, 0.1% SDS, and 42°C", and "0.2 × SSC, 0.1% SDS, and 65°C". Those skilled in the art are capable of appropriately obtaining the nucleotide sequences of homologs on the basis of the nucleotide sequences described in SEQ ID NOs: 1 to 26 mentioned above or symbols in other organisms.

Each subject protein other than the proteins having the specific amino acid sequences described above is also included in the subject protein of the present invention as long as the protein has high homology (usually 65% or more or 70% or more, preferably 80% or more, more preferably 90% or more, most preferably 95% or more) and has functions of the subject protein.

As used herein, the phrase "reducing expression or functions of a gene" means causing reduction in or loss of expression of a subject gene or a subject protein, or causing reduction in or loss of functions of a subject protein. Examples of the method for reducing expression or functions of a subject gene include disrupting the subject gene, suppressing transcription of the subject gene, degrading a transcript of the subject gene, inhibiting translation of the subject gene, inhibiting the interaction between the subject protein and another molecule, and inhibiting normal folding of the subject protein.

Specific approaches that achieve these methods include an approach involving genomic modification in a human or a non-human animal, and an approach involving no genomic modification. Examples of the approach involving no genomic modification include expression constructs, such as epigenetic regulators, inhibitory nucleic acid factors (miRNA, etc.), and RNA editing factors, which reduce expression or functions of a subject gene, and administration of arbitrary small molecules that bind to a subject protein. In a preferable example of the present invention, an approach involving modification of a subject gene is preferred from the viewpoint of also obtaining an animal fiber having a desired character from human or non-human animal progeny.

As used herein, the "modification" means artificially deleting, substituting, and/or inserting a nucleotide so that a predetermined nucleotide sequence is changed to another nucleotide sequence. The nucleotide may be a one-base nucleotide, an oligonucleotide, a long chain of nucleotides, or the like. Examples of the modification include missense mutation, frameshift mutation, nonsense mutation, and deletion of one or more amino acids. The genetically modified animal undergoes modification, which reduces expression or functions of a modified gene or protein, or disrupts the modified gene or protein.

As used herein, the "knockout" means modification that causes reduction in expression or functions of a subject gene, preferably disruption of the subject gene. The knockout is not limited as long as such modification occurs. A subject region may be modified, or an arbitrary region on the genome other than the subject region may be modified. As used herein, the "subject region" refers to a coding region of a subject gene and an arbitrary cis or trans element-encoding region that controls expression of the subject gene outside the coding region. The modification of an arbitrary region on the genome also encompasses insertion of an external gene that causes reduction in expression or functions of a subject gene or disruption of the subject gene, for example, modification that inserts an expression cassette for expressing a genome editing factor, an inhibitory nucleic acid factor, an RNA editing factor, an epigenetic regulator, or the like. Such an expression cassette does not have to cause reduction in expression or functions of a subject gene or disruption of the subject gene immediately after insertion, and may cause reduction or disruption after a lapse of a predetermined period.

In the present invention, the knockout can be modification of a portion or the whole of the subject region. The term "a portion" is a portion of the subject region and is a region having a length necessary for causing reduction in expression or functions of a gene or disruption of the gene. In the case of deleting or inserting a nucleotide from or to a subject region, frameshift can cause reduction in expression or functions of a gene or disruption of the gene, particularly, loss of expression or functions of the gene. Even in the absence of frameshift, nonsense mutation that gives rise to a stop codon by deletion, substitution, or insertion can cause reduction in expression or functions of a subject protein or disruption of the subject protein, particularly, loss of expression or functions of the subject protein. As for a gene having a plurality of exons, an arbitrary exon, for example, but not limited to, exon 1, 2, 3, 4, 5, or 6 can be modified. Preferably, exon 1 or exon 2 can be modified (frameshift mutation or nonsense mutation).

In the present invention, the knockout may be homozygous or heterozygous as long as the knockout can cause reduction in expression or functions of a gene or disruption of the gene. As used herein, the term "homozygous" means that chromosomes from both parents (both alleles) have modification and is also represented by [-/-] in the present specification. As used herein, the term "heterozygous" used for genetic modification means that chromosome from either parent (one allele) has the modification and is also represented by [-/+] or [+/] in the present specification. It can be understood that even the heterozygous knockout causes loss of functions of a gene as long as the knockout is dominant negative mutation; and other mutations are also capable of causing reduction in expression or functions as a whole as long as expression or functions are reduced at one allele. The modification, regardless of being homozygous or heterozygous, is inherited to a portion or the whole of progeny. Therefore, the progeny also has modification that causes reduction in expression or functions of a gene and is thus beneficial. In the present invention, the knockout is preferably homozygous knockout from the viewpoint of producing a phenotype and from the viewpoint of efficiently obtaining a homozygous knockout animal.

The modification method is not limited and can be performed by an arbitrary method such as genome editing or homologous recombination. Genome editing is preferred because of convenience and high modification efficiency. Examples of the genome editing include a method using a genome editing factor comprising nuclease capable of cleaving target double-stranded DNA and a DNA recognition component that binds to the nuclease. Examples of the genome editing include ZFN, TALEN, and CRISPR/Cas. For example, ZFN employs FokI (nuclease) and a zinc finger motif (DNA recognition component), TALEN employs FokI (nuclease) and a TAL effector (DNA recognition component), and CRISPR employs Cas (nuclease) and guide RNA (gRNA) (DNA recognition component). The nuclease for use in the genome editing can have nuclease activity, and DNA polymerase, recombinase, or the like may be used instead of the nuclease.

The nuclease is not limited, and CRISPR type I (type IA to IC, type I-D (Cas3', Cas5d, Cas6d, Cas7d, and Cas10d), and type I-E (Cas3, Cse1, Cse2, Cas7, Cas5, and Cas6)), type II (Cas9, etc.), type III, and type V (Cas12a (Cpf1, MAD2, MAD7, etc.)) can be used. In one aspect, CRISPR/Cas employs gRNA as a DNA recognition component. The gRNA may be single gRNA or may be dual gRNA of crRNA and tracrRNA. The genome editing does not necessarily require nuclease activity, and an arbitrary editing approach such as prime editing or Target-AID can be used (Anzalone et al., 2019 Nature vol 576, pages 149-157; and Nishimasu et al., Science 21 Sep 2018: Vol. 361, Issue 6408, pp. 1259-1262).

The target sequence of gRNA targeting a subject gene can be designed by use of a usual method. Such a design method is described in, for example, Naito et al., Bioinformatics. 2015 Apr 1; 31 (7): 1120-1123; and Park et al., Bioinformatics. 2016 Jul 1; 32 (13): 2017-2023. The gRNA can be prepared on the basis of these literatures and sequence information on the subject gene or the like.

The target sequence of gRNA targeting mouse Krt71 is not limited, and the sequences of SEQ ID NOs: 1 to 3 can be used. For other animal species, Krt71 gene can be disrupted using gRNA having a nucleotide sequence corresponding to the target sequence of gRNA.

The target sequence of gRNA targeting mouse Krt72 is not limited, and the sequences of SEQ ID NOs: 4 to 6 can be used. For other animal species, Krt72 gene can be disrupted using gRNA having a nucleotide sequence corresponding to the target sequence of gRNA.

The target sequence of gRNA targeting mouse Krt74 is not limited, and the sequences of SEQ ID NOs: 10 to 12 can be used. For other animal species, Krt74 gene can be disrupted using gRNA having a nucleotide sequence corresponding to the target sequence of gRNA.

The target sequence of gRNA targeting mouse KAP8-1 is not limited, and the sequences of SEQ ID NOs: 13 to 15 can be used. For other animal species, KAP8-1 gene can be disrupted using gRNA having a nucleotide sequence corresponding to the target sequence of gRNA.

The target sequence of gRNA targeting a mouse Tyr sequence is not limited, and the sequences of SEQ ID NOs: 19 to 21 can be used. For other animal species, KAP8-1 gene can be disrupted using gRNA having a nucleotide sequence corresponding to the target sequence of gRNA.

The target sequence of gRNA targeting a mouse Fgf5 sequence is not limited, and the sequences of SEQ ID NOs: 22 to 24 can be used. For other animal species, KAP8-1 gene can be disrupted using gRNA having a nucleotide sequence corresponding to the target sequence of gRNA.

Examples of the modification subject include embryos, somatic cells, germ cells, and individuals. In the case of introducing a genome editing factor to an embryo, the embryo modified by introduction can be transplanted into the uterus of an individual to obtain a non-human animal individual. For introduction to a somatic cell, an embryo obtained through nuclear transfer involving introducing the nucleus of the somatic cell modified by introduction to a denucleated egg cell can be transplanted into the uterus of an individual. In addition, a chimeric embryo prepared by implanting a cell derived from the modified embryo into another embryo may be transplanted into the uterus of an individual. For introduction to a germ cell, the germ cell modified by introduction can be directly used or transplanted to an individual, followed by fertilization (natural mating, artificial insemination, or *in vitro* fertilization, etc.) to obtain a non-human animal individual.

For introduction to an individual, a composition containing a genome editing factor in a form such as a virus vector may be administered directly to the individual to obtain a non-human animal having a modified cell. In this case, a character such as a thin fiber diameter of an animal fiber appears when the modification occurs at least in a cell constituting the hair follicle. Reduction in expression or functions of a subject gene can be caused in progeny thereof when the modification occurs in a germline. Whether the modification subject has undergone the modification can be determined by use of a usual method, for example, PCR, Southern blot, or genome sequencing.

An introduction method is not limited, and electroporation, lipofection, a calcium phosphate method, a dextran method, microinjection, iTOP (D'Astolfo et al., Cell. 2015; 161 (3): 674-690), a cell penetrating peptide (CPP), a virus vector, or a non-viral vector can be used. For an introduction to an embryo, electroporation is preferred because of high introduction efficiency. For introduction to an individual, a delivery approach using a virus vector or a non-viral vector is preferred. Examples of the virus vector include, but are not limited to, vectors based on adenovirus, adeno-associated virus (AAV), retrovirus, vaccinia virus, poxvirus, lentivirus, or herpes virus.

In one aspect of the present invention, an inhibitory nucleic acid may be administered to a non-human animal in addition to modifying a gene of the non-human animal. The inhibitory nucleic acid means a nucleic acid that inhibits expression of a target molecule, and includes an RNAi molecule, microRNA, a microRNA mimic, piRNA (piwi-interacting RNA), ribozyme, an antisense nucleic acid, BONAC nucleic acid (WO 2012/005368), and the like. The nucleic acid may include a modified nucleic acid and may be a hybrid molecule (gapmer, mixmer, etc.) of DNA and RNA.

The RNAi (RNA interference) molecule means an arbitrary molecule having RNAi activity. Examples thereof include, but are not limited to, siRNA (small interfering RNA) and shRNA (short hairpin RNA). The RNAi interference typically refers to a phenomenon in which target RNA is degraded in a sequence-specific manner, induced by a double-stranded nucleic acid molecule. The double-stranded nucleic acid molecule, when entering a cell, is cleaved by dicer depending on its length and then incorporated into RNA-induced silencing complex (RISC) containing argonaute (AGO) protein. The RISC recognizes target RNA by using an antisense strand (guide strand) having a sequence complementary to the target RNA as a guide, and cleaves this target RNA. siRNA is typically double-stranded RNA having a length of approximately 20 to 25 mer. shRNA is typically RNA obtained by linking two strands of siRNA via a hairpin.

A gene expression control mechanism based on the miRNA typically refers to sequence-specific degradation or translational suppression of RNA induced by the miRNA. Mature miRNA is incorporated into miRNA-induced silencing complex (miRISC) containing argonaute (AGO) protein and mainly directs the miRISC to mRNA complementary to a seed sequence (sequence from positions 2 to 8 from the 5' end). The miRISC suppresses translation from the mRNA while degrading 3'-terminal poly A tail of the mRNA and reducing the size of the mRNA, thereby promoting the degradation of the mRNA. A gene expression control mechanism based on the piRNA typically refers to sequence-specific degradation of RNA induced by the piRNA. The piRNA, when incorporated into piRNA-induced silencing complex (piRISC) containing Piwi protein which is a PIWI subfamily protein, is translocated into the nucleus where the piRISC suppresses transcription of a target gene in a sequence-specific manner. rasiRNA (repeat associated siRNA) also causes gene silencing mediated by Piwi protein.

The microRNA mimic is a nucleic acid molecule that mimics functions of endogenous microRNA and is well known in the art (e.g., van Rooij and Kauppinen, EMBO Mol Med. 2014; 6 (7): 851-64; and Chorn et al., RNA. 2012; 18 (10): 1796-804). The microRNA mimic may contain chemical modification, or its nucleotide sequence may be changed against endogenous microRNA.

The shRNA is a single-stranded RNA molecule comprising an antisense region and a sense region having complementarity to each other, and a loop region that intervenes between these regions. The antisense region and the sense region form a duplex region through pairing and assume a hairpin-shaped three-dimensional structure. The shRNA is cleaved by dicer in a cell to form a double-stranded siRNA molecule, which is in turn incorporated into RISC, causing RNA interference.

The shRNA is typically expressed from a nucleic acid construct encoding this shRNA, a plasmid containing this shRNA, or the like in a cell where the shRNA exerts its effect. The shRNA molecule may be delivered directly to a cell. These inhibitory nucleic acids can be appropriately prepared by methods known in the art on the basis of information on a target gene included in the present specification (Disney et al., Cold Spring Harb Perspect Biol. 2018 Nov 1; 10 (11); Naito and Ui-Tei, Front Genet. 2012; 3: 102; Mickiewicz et al., Acta Biochim Pol. 2016; 63 (1): 71-77; etc.). Since CRISPR/Cas13 can edit RNA, the genome editing factor can be used in gene silencing, as in the inhibitory nucleic acids.

In the method of the present invention, in the case of administering various compositions that cause reduction in expression or functions of a gene to non-human animals, the compositions may be administered through various routes that encompass oral and parenteral ones, for example, but not limited to, oral, transdermal, subcutaneous, intracutaneous, transmucosal, intravenous, intramuscular, local, rectal, intra-arterial, intraportal, intraventricular, intramyocardial, intra-articular, intranasal, intraperitoneal, tracheobronchial, intrabronchial, intra-alveolar, intrapulmonary, and intrauterine routes, and may be formulated into dosage forms suitable for each administration route. An arbitrary dosage form and formulation method known in the art can be appropriately adopted.

In the present invention, expression or functions of an additional arbitrary gene may be reduced or improved as long as expression or functions of at least one gene (subject gene) selected from one or more genes constituting the Krt or KAP family are reduced, or the at least one gene is disrupted. The additional gene can be a gene that imparts an animal fiber-related character or other arbitrary characters to a non-human animal or improves such a character. The gene, expression or functions of which are to be reduced, or the gene to be disrupted can be preferably, but not limited to, Fgf-5, Tyr, MSTN, ASIP, BCO2, CFTR, or the like, and these genes can be knocked out. The gene, expression or functions of which are to be improved can be preferably, but not limited to, Tβ4, VEGF (VEGF-A), AANAT, ASMT, or the like. For example, an expression cassette containing these genes may be knocked in. Expression or functions of a plurality of genes may be improved or reduced at the same time without inhibiting a desired character of the non-human animal of the present invention.

In an exemplary aspect, the non-human animal of the present invention further comprises reducing expression or functions of either or both of Fgf5 gene and Tyr gene, or disruption of the gene(s), in addition to reducing expression or functions of a subject gene.

The animal fiber length of the non-human animal is increased by reducing expression or functions of the Fgf5 gene, or disrupting the Fgf5 gene. The animal fiber of the non-human animal has white color by reducing expression or functions of the Tyr gene. Hence, in one aspect of the present invention, reduction in expression or functions of at least one gene selected from Krt and KAP can be combined with reduction in expression or functions of either or both of Fgf5 and Tyr to obtain a non-human animal having an animal fiber having a thin fiber diameter and a large fiber length (reduction in expression or functions of at least one gene selected from Krt and KAP, and Fgf5 gene), a non-human animal having an animal fiber having a thin fiber diameter and white color (reduction in expression or functions of at least one gene selected from Krt and KAP, and Tyr gene), a non-human animal having an animal fiber having a thin fiber diameter, a large fiber length, and white color (reduction in expression or functions of at least one gene selected from Krt and KAP, and Fgf5 and Tyr genes), or a non-human animal having an animal fiber having a large fiber length and white color (reduction in expression or functions of Tyr and Fgf5 genes). The method for reducing expression or functions of a subject gene, or disrupting the subject gene as described above in detail can be used as a method for reducing expression or functions of the Fgf5 and Tyr genes, or disrupting the genes.

It has heretofore been known that fiber lengths are increased in animals, such as mice, dogs, cats, and goats, having knockout in Fgf5 gene (Cell. 1994 Sep 23; 78 (6): 1017-25; Anim Genet. 2006 Aug; 37 (4): 309-15; Anim Genet. 2007 Jun; 38 (3): 218-21; and PLoS One. 2016 Oct 18; 11 (10): e0164640). It has also been known that animal fibers are white in animals, such as mice, rabbits, bovines, and minks, having knockout in Tyr gene (Mamm Genome. 2004 Oct; 15 (10): 749-58; Mamm Genome. 2000 Aug; 11 (8): 700-2; Mamm Genome. 2004 Jan; 15 (1): 62-7; and Anim Genet. 2008 Dec; 39 (6): 645-8). It is thus obvious that a character based on modification of a particular gene in a particular non-human animal species emerges as the same character in modification of orthologs of other animal species as long as the character is related to animal fibers. This is considered to also hold true for a gene such as Krt or KAP.

The non-human animal prepared by the method of the present invention is a genetically modified non-human animal having, for example, reduction in expression or functions of at least one gene selected from one or more genes constituting the Krt or KAP family, or disruption of the at least one gene, and has a thinner diameter of an animal fiber (fiber diameter) than that of the animal fiber of a syngeneic or allogeneic control non-human animal that does not have the genetic modification (e.g., before genetic modification). The sequence before genetic modification can be a sequence as illustrated with a registration number in the present specification. The fiber diameter typically refers to an average fiber diameter (particularly, an average fiber major axis). The control non-human animal typically refers to wild type. In one aspect of the present invention, the average fiber diameter of the non-human animal having modification of at least one gene selected from one or more genes constituting the Krt or KAP family is thinner by 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, or 20% or more than that of a syngeneic or allogeneic control non-human animal. The average fiber diameter of the non-human animal having the modification is thinner by, for example, 70% or less, 60% or less, 50% or less, 40% or less, 30% or less, 25% or less, 20% or less, 15% or less, or 10% or less than that of a syngeneic or allogeneic control non-human animal. The average fiber diameter of the non-human animal having the modification is thinner by 5 to 40%, 5 to 30%, 5 to 20%, 10 to 20%, 15 to 20%, 5 to 15%, 10 to 15%, or 5 to 10% than that of a syngeneic or allogeneic control non-human animal. The genetically modified animal having a 5 to 20%, 10 to 20%, 15 to 20%, 5 to 15%, 10 to 15%, or 5 to 10% thinner average fiber diameter than that of a syngeneic or allogeneic control non-human animal can be obtained by selection from among animals having the modification.

Decrease in fiber diameter can produce commercial value. For example, cashmere is graded depending on the characteristics of animal fibers including average fiber diameters (see Table 1). According to this table, 5% decrease in fiber diameter corresponds to decrease by 0.75 µm to 0.95 µm in average fiber diameter, which means that the grade elevates by one or two. Also, 20% decrease in fiber diameter means decrease from 19 µm as the largest diameter of grade 3 to 15.2 µm, which means that the grade elevates from grade 3 to top grade. The value of the modification technique that gives rise to this elevation in grade is considered drastic.

**[Table 1]**

| Table 1: Grade of cashmere | | | |
|---|---|---|---|
| # | Grade of quality | Average fineness (average diameter) | Ratio of unnecessary hair to cashmere |
| 1 | Top grade | 15.5 µm or less | up to 20% by weight |
| 2 | Grade 1 | 15.51-16.8 µm | up to 20% by weight |
| 3 | Grade 2 | 16.81-17.5 µm | 20.1-30.0 % by weight |
| 4 | Grade 3 | 17.51-19.0 µm | 30.1-60.0 % by weight |

The average fiber diameter can be measured, for example, by observing, under an optical or electron microscope, the diameters at the same sites of a plurality of animal fibers collected from the same location on body surface of a non-human animal at the same age (e.g., predetermined lengths from the hair root portions of animal fibers collected from the same location of a non-human animal, or diameters of predetermined lengths from the roots of animal fibers cut by the same method). Another measurement method can be a method for use as a testing method for wool fibers, for example, an arbitrary method, such as a projection microscope (IWTO1-8; see the standards of JIS L 1081 A), airflow (IWTO-28; see the standards of JIS L 1081 B), laser scan (see the standards of JIS L 1081 C), or an optical analyzer (IWTO-47; see the standards of JIS L 1081 D), using a plurality of animal fibers obtained from a non-human animal at the same age to measure an average fiber diameter of the animal fibers. The diameter of the animal fiber is preferably a major axis.

Thus, in one aspect, the present invention also provides a method for preparing an animal fiber having a thin average fiber diameter. In this aspect, the method can comprise, for example, one or two or more arbitrary steps of: raising a non-human animal; and cutting an animal fiber from the non-human animal, in addition to reducing expression or functions of at least one gene selected from one or more genes constituting the non-human animal Krt or KAP family.

Average fiber diameters are known to contribute to improvement in properties, such as texture, feeling to the skin, wear comfort, sheen, stretchiness, heat retention, and fast dryness, of fiber products. Thus, a fiber product prepared according to the present invention can be obtained as a fiber product improved in one or more properties selected from texture, feeling to the skin, wear comfort, softness, stretchiness, heat retention, fast dryness, and sheen.

In one aspect, the non-human animal prepared by the method of the present invention has smooth fiber surface as compared with a syngeneic or allogeneic control non-human animal. The "smooth fiber surface" specifically means that, for example, scales present on the surface of an animal fiber have a small scale thickness and/or a long scale interval. The "scales" are a squamous structure pineally arranged on the surface of an animal fiber. Each scale overlaps with another scale on the hair root side of the animal fiber, and this overlap gives rise to surface asperities of the animal fiber. A feather fiber has a structure called "nodes" similar to these scales. The "scales" of the present invention also encompass nodes of a feather fiber.

As used herein, the scale thickness is the thickness of a projection where a scale overlaps with another scale. A smaller scale thickness decreases the thickness of the projection. It can therefore be understood that, for example, a smaller average scale thickness produces an animal fiber "having smooth surface". In one aspect of the present invention, the scale thickness of the animal fiber of a non-human animal in which at least one gene selected from one or more genes constituting the Krt or KAP family is knocked out is significantly smaller than the average scale thickness of a syngeneic or allogeneic control non-human animal, particularly, wild type.

The average scale thickness can be measured, for example, by observing, under an optical or electron microscope, the scale thicknesses of scales residing at the same positions on a plurality of animal fibers collected from the same portion on body surface of a non-human animal at the same age (e.g., the scale thicknesses of scales residing in portions with predetermined lengths from the hair root portions of animal fibers collected from the same portion on body surface of a non-human animal, or portions with predetermined lengths from the roots of animal fibers cut by the same method). Since difference in average scale thickness can be visually evaluated, the measurement does not necessarily require appearing as a numeric value. For example, decrease in average scale thickness can be evaluated by comparing electron microscope images of the animal fiber of the non-human animal prepared by the method of the invention and the animal fiber of a syngeneic or allogeneic control non-human animal, and using an index such as shadows created by scales or contours of scales. The "small scale thickness" refers to light shadows created by scales or indistinct contours of scales as compared with the animal fiber of a syngeneic or allogeneic control non-human animal. When the average scale thickness can be measured as a numeric value, the scale thickness is smaller by, for example, 98% or less, 96% or less, 95% or less, 93% or less, 90% or less, 85% or less, 80% or less, or 75% or less, preferably 70% or less, more preferably 65% or less, further preferably 30% or less, still further preferably 20% or less, than that of the animal fiber of a syngeneic or allogeneic control non-human animal.

The sheen can be evaluated by visually observing portions residing at the same positions on a plurality of animal fibers collected from the same portions on body surface of a non-human animal and a control at the same age (e.g., portions with predetermined lengths from the hair root portions of animal fibers collected from the same portions on body surface of non-human animals, or portions with predetermined lengths from the roots of animal fibers cut by the same method). The evaluation can be conducted by evaluation panelists composed of a plurality of persons. The evaluation panelists can evaluate the sheen of the animal fibers on the basis of predetermined scores related to good or poor sheen. In the scores, for example, excellent sheen can be given 5, average sheen or sheen equivalent to the control can be given 3, poor sheen can be given 1, sheen between the highest score and the average can be given 4, and sheen between the average and the lowest score can be given 2.

In one aspect of the present invention, the scale interval of the animal fiber of a non-human animal in which at least one gene selected from one or more genes constituting the Krt or KAP family is knocked out is significantly longer than that of a control non-human animal, particularly, wild type.

The scale interval is the major axis of each scale. The longer the major axis of each scale residing on the surface of an animal fiber, the smaller the number of scales present at a predetermined fiber length. For example, an animal fiber having scales having a scale interval of 25 µm has four scales per 100 µm, whereas an animal fiber having scales having an average scale interval of 50 µm has only two scales per 100 µm and therefore decreases the frequency of asperities resulting from the scales. It can thus be understood that a longer scale interval, particularly, a longer average scale interval, offers "smooth fiber surface".

The average scale interval can be measured, for example, by observing, under an optical or electron microscope, the scale intervals of the same portions of a plurality of animal fibers collected from the same portion on body surface of a non-human animal at the same age (e.g., the scale intervals of scales residing in portions with predetermined lengths from the hair root portions of animal fibers collected from the same portion on body surface of a non-human animal, or portions with predetermined lengths from the roots of animal fibers cut by the same method).

The "long scale interval" means that, for example, the average scale interval thus measured is longer by 102% or more, 105% or more, 110% or more, 120% or more, 130% or more, 140% or more, 150%, or 160% or more, preferably 170% or more, more preferably 180% or more, for example, longer by 105% to 180%, than that of a control non-human animal.

It has heretofore been known that scales on animal fibers are responsible for entanglement or shrinkage that causes pilling (pill formation) or felting in fiber products of textile or knit. Furthermore, the scales are not only responsible for prickly stimuli at the time of wearing fiber products but responsible for impairing the sheen, softness, smoothness, and the like of the products themselves. In order to cope with these problems, the scales are subjected to peeling treatment by a chemical or physical method such as an oxidizing agent, an enzyme, or plasma discharge, or a method of coating asperities of the scales with a coating resin is adopted.

Thus, in one aspect, the present invention provides a method for preparing a fiber product comprising an animal fiber having one or more properties selected from unlikeliness to cause pilling and/or felting, high softness, strong smoothness, few stimuli, strong sheen, and no shrinkage. In this aspect, the method can comprise one or two or more arbitrary steps, in addition to reducing expression or functions of at least one gene selected from one or more genes constituting the non-human animal Krt or KAP family, cutting an animal fiber from a non-human animal, and producing a fiber product from the cut animal fiber.

In this aspect, the method preferably comprises no step of performing scale peeling treatment or scale coating treatment. In one aspect, an animal fiber product obtained from the non-human animal prepared by the method of the present invention is unlikely to cause pilling and/or felting and contribute to improvement in properties such as softness, no stimulus (texture, feeling to the skin, and wear comfort), and shrink resistance. Therefore, the non-human animal can be preferably used for production of a fiber product improved in these properties. Particularly, pilling and/or felting occurs more easily with decrease in average fiber diameter. The non-human animal is preferably used in the preparation of an animal fiber such as a cashmere, mohair, Angora, Cashgora, yak, ibex, guanaco, qiviuk, chiru, raccoon dog, raccoon, mink, sable, fox, possum, opossum, beaver, seal, nutria, ferret, vicugna, wild duck, goose, duck, or ostrich fiber having a thin average fiber diameter.

In one aspect of the present invention, the animal fiber can be raw hair of an animal. The raw hair (particularly, fur) can generally comprise guard hair and vellus hair.

In one aspect of the present invention, the animal fiber contains unnecessary hair only at a proportion of preferably 60% by weight or less, 50% by weight or less, 40% by weight or less, 30% by weight or less, 25% by weight or less, 20% by weight or less, or 15% by weight or less. The unnecessary hair means, for example, guard hair for fur (e.g., a goat (e.g., cashmere, mohair, Angora, and Cashgora). The animal fiber preferably contains 40% by weight or more, 50% by weight or more, 60% by weight or more, 70% by weight or more, 80% by weight or more, or 85% by weight or more of undercoat. The unnecessary hair is guard hair for a goat (e.g., cashmere, mohair, Angora, and Cashgora). The goat (e.g., cashmere, mohair, Angora, and Cashgora) has vellus hair (also called undercoat or underfur) in addition to the guard hair.

The average fiber diameter of the animal fiber is thinner by 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, or 20% or more than that of the animal fiber of a syngeneic or allogeneic control non-human animal. The average fiber diameter of the animal fiber is thinner by 50% or less, 40% or less, 30% or less, 25% or less, 20% or less, 15% or less, or 10% or less than that of the animal fiber of a syngeneic or allogeneic control non-human animal. The average fiber diameter of the animal fiber is thinner by 5 to 40%, 5 to 30%, 5 to 20%, 10 to 20%, 15 to 20%, 5 to 15%, 10 to 15%, or 5 to 10% than that of the animal fiber of a syngeneic or allogeneic control non-human animal. Such a fiber may be obtained by selecting such an animal fiber from animal fibers cut from the genetically modified animal of the present invention, or may be obtained by cutting (without or through selection) from the genetically modified animal of the present invention having such a fiber system.

The animal fiber obtained from the genetically modified animal has a small product content of proteins encoded by the modified Krt gene and KAP gene in the genetically modified animal compared with a syngeneic or allogeneic control non-human animal. In a preferable aspect, the animal fiber obtained from the genetically modified animal is substantially free from proteins encoded by the modified Krt gene and KAP gene in the genetically modified animal.

In one aspect, the non-human animal prepared by the method of the present invention has a small average fiber diameter and smooth fiber surface as compared with a control non-human animal. Thus, a fiber product that is unlikely to cause pilling and/or felting and can maintain quality and is wearable over a long period can be provided in spite of a thin average fiber diameter.

The animal prepared by the method of the present invention has a thin average fiber diameter of the whole body hair. Therefore, the animal fiber obtained from this animal can be produced by omitting a selection step of selecting only thin hair. This approach is economically rational.

If the animal fiber of the prepared non-human animal differs in average fiber diameter among individuals, an individual having a small average fiber diameter is selected and then the animal fiber may be recovered from the selected individual. As a result, an animal fiber having a small average fiber diameter can be mainly recovered.

In one aspect, raw hair of an animal fiber can be obtained from the non-human animal prepared by the method of the present invention. The present invention provides a method for obtaining produced hair from the raw hair of the animal fiber. The method for obtaining produced hair from the raw hair of the animal fiber can comprise: loosening the raw hair; removing dirt and dandruff; washing the obtained raw hair (e.g., which includes washing with a neutral detergent, washing with hot water, and washing with water); and separating vellus hair and guard hair that coexist in the raw hair to isolate the vellus hair (produced hair processing). In the step of produced hair processing, it is reported that enhancing a yield while minimizing a guard hair content is important. The produced hair processing is an important step for the processing of cashmere. The produced hair can be subjected, if necessary, to dyeing (e.g., by a low-temperature dyeing method). The undyed produced hair or raw hair or the dyed produced hair or raw hair can be processed into a woolen yarn by spinning, and the woolen yarn can be processed into a twisted yarn or a worsted yarn by combining yarns. Typically, the woolen yarn, the twisted yarn, or the worsted yarn is packed and shipped. The woolen yarn, the twisted yarn, or the worsted yarn is processed into distinctive textile depending on a purpose. The present invention can also provide produced hair, a woolen yarn, a twisted yarn, a worsted yarn, and textile obtainable from the animal fiber. The produced hair, the woolen yarn, the twisted yarn, the worsted yarn, and the textile obtained in the present invention are derived from the animal fiber described above and therefore have the average fiber diameter and/or the scale interval or the scale thickness of the animal fiber described above. The process of the produced hair is often not carried out in the preparation of general fur (e.g., mink or sable fur) and however, can be performed in the preparation of high-quality products (particularly, high-grade products) such as cashmere.

In one aspect, the animal fiber after produced hair processing (e.g., "tops") has a lower content of unnecessary hair (e.g., guard hair) than that of raw hair. The animal fiber after produced hair processing contains only 60% by weight or less, 50% by weight or less, 40% by weight or less, 30% by weight or less, 20% by weight or less, 15% by weight or less, 10% by weight or less, or 5% by weight or less of unnecessary hair. When the content of unnecessary hair in raw hair is defined as A, the content (B) of unnecessary hair in the animal fiber after produced hair processing can be 95% by weight or less, 90% by weight or less, 80% by weight or less, 70% by weight or less, 60% by weight or less, 50% by weight or less, 40% by weight or less, 30% by weight or less, 20% by weight or less, 15% by weight or less, 10% by weight or less, or 5% by weight or less of A. When the content of unnecessary hair in raw hair is defined as A, the content (B) of unnecessary hair in the produced hair can be, for example, 5% by weight to 90% by weight, 5% by weight to 60% by weight, 5% by weight to 30% by weight, or 5% by weight to 20% by weight. Thus, the content of unnecessary hair **(e.g.,** guard hair) in the woolen yarn, the twisted yarn, and the worsted yarn is also only 60% by weight or less, 50% by weight or less, 40% by weight or less, 30% by weight or less, 20% by weight or less, 15% by weight or less, 10% by weight or less, or 5% by weight or less. When the content of unnecessary hair in raw hair is defined as A, the content (C) of unnecessary hair in the woolen yarn, the twisted yarn, and the worsted yarn can be 95% by weight or less, 90% by weight or less, 80% by weight or less, 70% by weight or less, 60% by weight or less, 50% by weight or less, 40% by weight or less, 30% by weight or less, 20% by weight or less, 15% by weight or less, 10% by weight or less, or 5% by weight or less, of A. As a matter of course, the animal fiber, the woolen yarn, the twisted yarn, and the worsted yarn thus obtained contain a component based on the gene mutation described above. Specifically, the animal fiber, the woolen yarn, the twisted yarn, and the worsted yarn thus obtained contain only a smaller amount (e.g., only 50% by weight or less, 40% by weight or less, 30% by weight or less, 25% by weight or less, 20% by weight or less, 15% by weight or less, 10% by weight or less, 5% by weight or less, 3% by weight or less, 2% by weight or less, or 1% by weight or less) of a product of the modified gene than that of wild type, contain no product (e.g., protein) of the disrupted gene, or contain a peptide molecule (peptide molecule having reduction in or loss of its functions) encoded by the disrupted gene. Thus, the resulting animal fiber, woolen yarn, twisted yarn, and worsted yarn have a component content (particularly, contained protein ratio) different from that of syngeneic or allogeneic non-human control animal. The animal fiber, the woolen yarn, the twisted yarn, and the worsted yarn thus obtained are capable of having the thin diameter (major axis) defined above as compared with a syngeneic or allogeneic non-human control animal that does not have the gene disruption. These characteristics can be common characteristics of at least fur and the yarns derived from the fur.

As used herein, the "fiber product" is not limited as long as the fiber product comprises the animal fiber obtained from the animal prepared by the method of the present invention. The fiber product includes any form such as slivers, yarns (including woolen yarns and worsted yarns), fiber sheets, textile, knit, fabrics, cloth, unwoven fabrics, multi-axis fiber sheets, and fur, and clothing (final product) obtained therefrom, such as coats, sweaters, stoles, shawls, scarfs, shirts, pants, suits, blazers, skirts, blankets, lap robes, underwear, socks, and gloves. The fiber product also has the features (e.g., thickness, scale interval, scale thickness, unnecessary hair content, and component content) of the animal fiber after produced hair processing, the woolen yarn, the twisted yarn, or the worsted yarn.

The fiber product may contain no fiber other than the animal fiber or may contain a fiber other than the animal fiber. The fiber product may be mixed with, for example, a fiber of an animal of different species, a fiber derived from a plant, or a synthetic fiber or may contain a fiber of an animal of different species, a fiber derived from a plant, or a synthetic fiber. Examples of the fiber other than the animal fiber contained in the fiber product can include natural fibers such as cocoon fibers, spider fibers, silk, cotton, linen, and flax, synthetic fibers such as polyester, polyamide, acryl, polyethylene, and polypropylene, and semisynthetic fibers such as viscose rayon and cuprammonium rayon.

### Examples

Hereinafter, the present invention will be described in detail with reference to Examples. However, the contents of the present invention are not limited by these examples.

### Example 1: Mass spectrometry of animal fibers of mouse, cashmere goat, and sheep

### (1) Preparation of sample

Animal fibers of a wild-type mouse (C57BL/6N lineage; purchased from Japan SLC, Inc.; the same holds true for the description below), cashmere goat, and sheep were each added to an 8 M urea solution and dissolved by ultrasonication. To this solution, 0.26 M DTT was added, and the mixture was incubated at 37°C for 1 hour to reduce proteins in each sample. Then, 0.22 M iodoacetamide (IAA) was added to the sample, and the mixture was incubated at 37°C for 30 minutes to alkylate the proteins. Acetone precipitation was performed for precipitating and purifying the proteins from the sample. For the acetone precipitation, TCA/acetone (10% TCA in acetone) was first added in an amount of 10 times the amount of the sample and stirred. The sample was left standing overnight at -20°C and then centrifuged at 15000 × g at 4°C for 10 minutes. After removal of a supernatant, pellets were washed with a small amount of cold acetone and centrifuged, followed by the removal of a supernatant. After volatilization of acetone, the sample was redissolved by the addition of a 0.5 M urea solution.

A 0.2 mg/ml solution of trypsin in acetic acid was added to the sample, and the mixture was incubated overnight at 37°C for peptization. After the completion of incubation, the reaction was terminated by the addition of formic acid (10%). The peptized sample was desalted and concentrated using GL-Tip(R) SDB chip (GL Sciences Inc., Cat No. 7820-11200), followed by peptide concentration measurement (Pierce Quantitative Colorimetric Assay, Thermo Fisher Scientific Inc., Cat No. 233275).

### (2) Mass spectrometry and data analysis

Each peptide sample thus obtained was analyzed by use of mass spectrometry (LC-MS/MS). An ultrahigh-resolution Fourie transform mass spectrometry apparatus (Orbitrap Fusion) (Thermo Fisher Scientific Inc., Cat NO. IQLAAEGAAPFADBMBHQ) was used in LC measurement.

As a result, most of major proteins constituting the animal fibers were found common among the mouse, the cashmere goat, and the sheep. Animal fibers are formed by keratinization through differentiation and proliferation of hair matrix cells and the like residing in the hair follicle. Therefore, the commonality of constituents of the animal fibers reflects the commonality of differentiation, proliferation, and keratinization processes of hair matrix cells and the like, i.e., hair growth mechanisms, among animals. Accordingly, the animals are predicted to have common hair growth mechanisms as a whole. Animal fiber (body hair) formation is considered widely common among various animal species. For example, the action of Fgf5 is common among various species including a dog, a cat, a mouse, and a human and is also common to a cashmere goat (X. Wang et al., Plos One, 11 (10): e0164640, 2016). Fgf5 is an inhibitor for hair elongation and is expressed in the external root sheath so as to control the start of catagen in a hair growth cycle (J. M. Heabert et al., CELL, 78: 1017-1025, 1994).

### Example 2: Preparation of knockout mouse

In the description below, Krt71, Krt72, Krt73, Krt74, KAP8-1, KAP8-2, Tyr, and Fgf-5 were selected from many genes considered to participate in common hair growth mechanisms, regardless of animal species. Disruption of these genes was individually attempted.

### (1) Preparation of crRNA, tracrRNA and Cas9 protein

crRNA (Alt-R(TM), CRISPR-Cas9 crRNA) against mouse Krt71, Krt72, Krt73, Krt74, KAP8-1, KAP8-2, Tyr, or Fgf-5, tracrRNA (Alt-R(TM) CRISPR-Cas9 tracrRNA, 5 nmol, Cat# 1072532), and Cas9 protein (Alt R(TM) S.p. Cas9 Nuclease V3, 100 µg Cat# 1081058) were purchased from Integrated DNA Technologies, Inc. ((hereinafter, referred to as IDT), USA). Three types of crRNA sequences were designed for each target gene, and each obtained crRNA was added at 1 µg/µl to Opti-MEMI (Thermo Fisher Scientific Inc., Cat# 31985062) and dissolved therein. The nucleic acid sequences of DNA targeting sites in the crRNA sequences are as described below. These three types of crRNA sequences can target three different locations, and it is expected that mutations can be introduced to three locations at maximum for each gene.

Sequences of target sites of crRNA for Krt71 KO (T is changed to U on RNA)
   Krt71 crRNA1; CCTACCGGGCAGGAGGCAAA (SEQ ID NO: 1)
   Krt71 crRNA2; AGCGGGAAGAACGGAGGTTT (SEQ ID NO: 2)
   Krt71 crRNA3; ACCTGATGGATACCGCCAGG (SEQ ID NO: 3)
Sequences of target sites of crRNA for Krt72 KO (T is changed to U on RNA)
   Krt72 crRNA2; GTCATCTCAGGCAGGCTCAG (SEQ ID NO: 4)
   Krt72 crRNA3; AGAAGCCTGTTCTGTGTGGG (SEQ ID NO: 5)
   Krt72 crRNA4; CGGGATAGAGGGTCAACTGG (SEQ ID NO: 6)
Sequences of target sites of crRNA for Krt73 KO (T is changed to U on RNA)
   Krt73 crRNA1; TCTTACCGGGCAGCCGGCAA (SEQ ID NO: 7)
   Krt73 crRNA2; AGCGGGCGCACAGGGGGATA (SEQ ID NO: 8)
   Krt73 crRNA3; AATCCATCCTTGTGCCTGCC (SEQ ID NO: 9)
Sequences of target sites of crRNA for Krt74 KO (T is changed to U on RNA)
   Krt74 crRNA1; GATTCCCTCCAAGACTGTAG (SEQ ID NO: 10)
   Krt74 crRNA2; AGCTTTGGATATGGGTATGG (SEQ ID NO: 11)
   Krt74 crRNA3; TCTGTCTGGGGGCATCTACC (SEQ ID NO: 12)
Sequences of target sites of crRNA for KAP8-1 KO (T is changed to U on RNA)
   KAP8-1 cRNA1; GTCTTCCCAGGATGCTACTG (SEQ ID NO: 13)
   KAP8-1 cRNA2; CAGCCAACACTGTACCCCAG (SEQ ID NO: 14)
   KAP8-1 cRNA3; GGTAGCACCTACTCTCCAGT (SEQ ID NO: 15)
Sequences of target sites of crRNA for KAP8-2 KO (T is changed to U on RNA)
   KAP8-2 cRNA1; GTAGCTGCCGTAGTAGCTCA (SEQ ID NO: 16)
   KAP8-2 cRNA2; GGCTCTGGCATCCGAGGCTT (SEQ ID NO: 17)
   KAP8-2 cRNA3; GGAGGTTACGGATATGGATC (SEQ ID NO: 18)
Sequences of target sites of crRNA for Tyr KO (T is changed to U on RNA)
   Tyr crRNA1; TGCCAACAAGTTCTTAGAGG (SEQ ID NO: 19)
   Tyr crRNA2; GGTCATCCACCCCTTTGAAG (SEQ ID NO: 20)
   Tyr crRNA3; CAGTGCTCAGGCAACTTCAT (SEQ ID NO: 21)
Sequences of target sites of crRNA for Fgf5 KO (T is changed to U on RNA)
   Fgf5 crRNA1; CCCAAGCGCTGTGGATCAGG (SEQ ID NO: 22)
   Fgf5 crRNA2; ACGTCGCGCTACTTCTGCCC (SEQ ID NO: 23)
   Fgf5 crRNA3; GTTTCCAGTGGAGCCCTTCG (SEQ ID NO: 24)

### (2) Electroporation

C57BL/6N was purchased from Japan SLC, Inc. A sperm mass was recovered from the epididymis of male C57BL/6N. The sperm mass was incubated in FERTIUP(TM)-sperm preculture medium (Kyudo Co., Ltd. (hereinafter, referred to as Kyudo), Japan). An ovum mass was recovered into CARD mHTF medium (Kyudo) from female C57BL/6N in which ultra-superovulation was induced with CARD HyperOva(TM) (Kyudo). The sperm suspension was added to the medium containing the ovum for *in vitro* fertilization. After semination for 3 hours, the ovum was transferred three times to CARD KSOM medium (Kyudo) where the ovum was washed.

An electrode (BEX Co., Ltd. (hereinafter, referred to as BEX), Japan, Cat# LF501PT1-10) connected to GenomeEditor (BEX, Cat# GEB15) was loaded on a stereomicroscope. The fertilized eggs cultured in CARD KSOM medium were washed twice with Opti-MEMI to remove a serum-containing medium. Subsequently, the fertilized eggs were arranged in a gap of the electrode filled with Duplex Buffer (IDT) solution (5 µl) containing three types of crRNA (66 ng/µl) targeting each gene described above, tracrRNA (100 ng/µl), and Cas9 protein (100 ng/µl), and electroporated. The electrical conditions involved 25 V (3msec pulse + 97msec interval) three times each for one polarity and opposite polarity.

Immediately after electroporation, the fertilized eggs were recovered from the electrode and washed once with M2 medium and twice with CARD KSOM medium. Subsequently, the fertilized eggs were cultured until a two-cell stage in CARD KSOM medium at 37°C in a 5% CO₂ incubator.

### Example 3: Confirmation of gene mutation in knockout mouse

An embryo at the two-cell stage was transplanted to the oviducts of a pseudopregnant mouse of 0.5 days after mating. After 3 weeks from delivery, a portion of the ears was collected, treated in 90 µl of 50 mM NaOH at 98°C for 10 minutes, and then neutralized with 10 µl of 1 M Tris HCl (pH 8.0) to collect a genomic DNA solution. In order to examine mutations in each gene by CRISPR/Cas9, a genomic region adjacent to each target sequence was amplified by PCR using the following specific primers.

Primer set for confirmation of Krt71 KO
   mKrt71_Miseq_Fw; 
TCGTCGGCAGCGTCAGATGTGTATAAGAGACAGATCTACCTTCCTCCTGCAC
CT (SEQ ID NO: 25)
   mKrt71_Miseq_Rv; 
GTCTCGTGGGCTCGGAGATGTGTATAAGAGACATTGTTCAGAGCCTTGATCT
GCT (SEQ ID NO: 26)
Primer set for confirmation of Krt72 KO
   Krt72 N-Fw; AATTAGCTGACTCCATCCTGCC (SEQ ID NO: 27)
   Krt72 N-Rv; CCTTGATCTGCTCCCTCTCTTG (SEQ ID NO: 28)
   Krt72-Miseq_Fw; 
TCGTCGGCAGCGTCAGATGTGTATAAGAGACAGCCAGGAGTGCAGCTGTATC
C (SEQ ID NO: 107)
   Krt72-Miseq_Rv; 
GTCTCGTGGGCTCGGAGATGTGTATAAGAGACAGACATTGAGTGGGGCCAGA
AG (SEQ ID NO: 108)
Primer set for confirmation of Krt73 KO
   mKrt73_Miseq_Fw; 
TCGTCGGCAGCGTCAGATGTGTATAAGAGACAGCTGTCTGTGGCTTCTTCAG
GAT (SEQ ID NO: 20)
   mKrt73_Miseq_Rv; 
GTCTCGTGGGCTCGGAGATGTGTATAAGAGACAGCGAATTTGTTGTTCAGAG
CCT (SEQ ID NO: 30)
Primer set for confirmation of Krt74 KO
   mKrt74_Miseq_Fw; 
TCGTCGGCAGCGTCAGATGTGTATAAGAGACAGGTTGGCAACTGAACTTCAG
GTC (SEQ ID NO: 31)
   mKrt74_Miseq_Rv; 
GTCTCGTGGGCTCGGAGATGTGTATAAGAGACAACTTGTCGTTCAGAGCCTT
GAT (SEQ ID NO: 32)
Primer set for confirmation of KAP8-1 KO
   Krtap8-1 Fw; AAGACCAAGCGCTTTTGAAGTC (SEQ ID NO: 33)
   Krtap8-1 Rv; AGGTGTCACAAAGCCTTCATGA (SEQ ID NO: 34)
   KAP8-1-Miseq_Fw; 
TCGTCGGCAGCGTCAGATGTGTATAAGAGACAGAAGAATGAAAATGAGGGCC
TTGTG (SEQ ID NO: 35)
   KAP8-1-Miseq_Rv; 
GTCTCGTGGGCTCGGAGATGTGTATAAGAGACAGAGGTGTCACAAAGCCTTC
ATGA (SEQ ID NO: 36)
Primer set for confirmation of KAP8-2 KO
   KAP8-2_Miseq_Fw; 
TCGTCGGCAGCGTCAGATGTGTATAAGAGACAGACAACGGAAGGGTATGTTC
ATGA (SEQ ID NO: 37)
   KAP8-2_Miseq_Rv; 
GTCTCGTGGGCTCGGAGATGTGTATAAGAGACATTTCTTCTTGACTTGATTC
ACAGTCAG (SEQ ID NO: 38)
Primer set for confirmation of Tyr KO
   Tyr Fw; CCAGGGGTTGCTGGAAAAGA (SEQ ID NO: 39)
   Tyr Rv; TCTTTTCGGAGACACTCAAATCA (SEQ ID NO: 40)
Primer set for confirmation of Fgf5 KO
   Fgf5 Fw; GAGGCTATGTCCACCCTGTG (SEQ ID NO: 41)
   Fgf5 Rv; GTCCCTCCCAGAACAGGTTT (SEQ ID NO: 42)

A PCR amplification product of each gene was purified and analyzed for the DNA sequence of a target site using a next-generation sequencer Miseq (Illumina, Inc.) and commissioned sequence service provided by Eurofins Genomics K.K. (Japan).

As a result, one to three knockout mice having mutated sequences of Figures 1-1 to 8 were obtained for each gene. In Figures 1-1 to 8, a wild-type sequence (exon 1) was compared with mutated sequences (exon 1) in the knockout mice, and sequences deleted in the knockout mice are highlighted, insertion is underlined, and substitution is indicated by boldface.

All the obtained mutations were confirmed to be gene disruption ascribable to frameshift of each gene.

### Example 5: Confirmation of fineness of knockout animal fiber under scanning electron microscope

Awl hair of 3-week-old wild-type and Krt71, Krt72, Krt73, Krt74, KAP8-1, or KAP8-2 knockout mice was observed. A cross section at 2 mm from the tip of the mouse awl hair was observed at 3000× under a scanning electron microscope, and a major axis was measured. Specifically, embedding, cutting, and fixation were performed by the following procedures, followed by observation under a scanning electron microscope.

### [Photographing conditions]

Acceleration voltage: 5 kV (high-magnification observation mode)
WD: 20 mm
Observation angle: 0° (parallel with hair)
Observation magnification: 3000×

### [Fixation conditions]

Each awl hair (hereinafter, also referred to as a sample) was placed in a silicone mold with a 1 mm groove, and an ultraviolet-curable resin was added dropwise and poured into the mold. The position of the sample was adjusted so as not to come into contact with the wall surface of the mold. Then, the sample is irradiated for 3 hours or longer under 10 W black light. After confirmation of curing, extra resin was scraped off.

### [Preparation of cross section]

While the sample embedded in the resin was observed under a stereomicroscope, the hair was cut at a location of 2 mm from the tip using a one-blade razor. The position of 2 mm from the tip of the hair was measured and determined using a metal rule of the first grade of JIS standards. An x-mark was put on the razor used in cutting, and its use was prohibited in subsequent cross section preparation.

The hair root side was used for an observation sample, and the tip side of the hair is separately stored as a preliminary observation sample. Both the main and preliminary samples were numbered and stored in hermetically sealed plastic cases except when necessary.

### [Sputtering procedure]

The observation sample was fixed to a sample table that permitted observation at a right angle under an electron microscope. Fine Au particles were applied thereto for 120 seconds using a Au sputtering apparatus (JFC-1200FINE COATER (manufactured by JEOL Ltd.)) (theoretical value of a Au film thickness: approximately 20 nm).

### [Procedure of observation under electron microscope]

The sample was observed under an electron microscope (VHX-D510 (manufactured by Keyence Corp.). The direction of observation was fixed to a position parallel with the embedding resin, and the major axis of the sample was measured after photographing. The photographing conditions were as described below.

### [Photographing conditions]

Acceleration voltage: 5 kV (high-magnification observation mode)
WD: 8 mm
Observation angle: 0° (parallel with embedding resin)
Observation magnification: 3000×

The results are shown in Figure 10. At least 10 hair samples per individual were collected from the wild-type and knockout (KO) mice and subjected to observation. The fiber diameter (major axis) of the hair was normalized with the average fiber diameter of the hair of the wild type of the same age of week obtained at the same time.

As shown in Figure 10, a decreased fiber diameter of the hair of each of the Krt71, Krt72, Krt74, and KAP8-1 knockout mice was confirmed with reproducibility. Particularly, a decreased fiber diameter was found in a plurality of mutants differing in specific pattern of disruption, suggesting that the disruption of these genes contributes to decrease in fiber diameter. The tendency of this decrease in fiber diameter was not changed regardless of whether the observation was made under an optical microscope and whether fixation was performed for electron microscopy. It was also revealed that among the knocked-out genes, Krt72 and KAP8-1 most strongly decreased the fiber diameter. Particularly, more than 10% decrease in fiber diameter was observed in Krt72 KO, and approximately 20% decrease in fiber diameter was observed in KAP8-1 KO.

### Example 6: Observation of scale structure of knockout animal fiber under scanning electron microscope

The side of a portion of 2 mm from the tip of awl hair was photographed for all prepared knockout mice (3 weeks old) at 1200× under a scanning electron microscope. Fine Au particles were applied thereto for 60 seconds using a Au sputtering apparatus (DII-29010SCTR Smart Coater, manufactured by JEOL Ltd.) (theoretical value of a Au film thickness: approximately 5 nm). Fixation was not particularly performed.

### [Photographing conditions]

Acceleration voltage: 10 kV
WD: 20 mm
Observation magnification: 1200×

In order to measure the scale interval of the hair, scale widths were measured in each knockout mouse on the basis of the image of the photographed hair.

The results were as shown in Figure 11. As seen from Figure 11, the hair of the Krt72 knockout mouse and the hair of the KAP8-1 knockout mouse had an increased scale interval and a decreased number of scales contained in hair with a given length, as compared with the wild type. As is also evident, the hair of each knockout mouse had a decreased scale thickness, indistinct contours of scales, and smooth hair as a whole. The hair of the Krt72 knockout mouse was confirmed to have drastically smooth texture in the form of a hair bundle as compared with the wild type. This is presumably due to decrease in fiber diameter as well as increase in scale interval and decrease in scale thickness.

### Example 7: Observation of Fgf-5 knockout animal fiber

Awl hair was collected from each of 3-week-old wild-type and Fgf-5 knockout mice, and its length was measured. The results are shown in Table 10. One mouse was used for each type. n represents the number of hairs used in measurement, and mm that indicates the length of hair represents an average length thereof. "WT" represents wild type.

**[Table 2]**

| Table 2: Length of awl hair in Fgf-5 knockout animal | | |
|---|---|---|
| | Length of hair (mm) | |
| WT (n =9) | | 6.2 |
| Fgf-5 (n =6) | | 9.6 |

The hair length of the Fgf-5 knockout mouse was increased by 55% as compared with the wild type.

Fgf-5 and at least one gene selected from the group consisting of the Krp family or the KAP family are disrupted in combination. The resulting animal has a thin fiber diameter and produces a long animal fiber.

### Example 8: Observation of Tyr knockout animal fiber

Figure 12 shows photographs of a 3-week-old wild-type mouse (C57BL/6N lineage; black) and Tyr knockout mouse thereof. It was possible to confirm that the body hair of the wild type colored black, whereas the Tyr knockout mouse had white hair (see Figure 12).

Tyr and at least one gene selected from the group consisting of the Krp family or the KAP family are disrupted in combination. The resulting animal has a thin fiber diameter and produces a white animal fiber.

Fgf-5, Tyr, and at least one gene selected from the group consisting of the Krp family or the KAP family are disrupted in combination. The resulting animal has a thin fiber diameter and produces a long white animal fiber.

### Example 9: Analysis of animal fiber of goat having Krt family gene disruption

In this Example, a goat having gene disruption of Krt72 was prepared and analyzed for the diameter of an animal fiber thereof. The animal fiber used was undercoat.

### (1) Preparation of crRNA, tracrRNA and Cas9 protein

crRNA (Alt-R(TM), CRISPR-Cas9 crRNA) against goat KAP8-1, tracrRNA (Alt-R(TM) CRISPR-Cas9 tracrRNA, 5 nmol, Cat# 1072532), Cas9 electroporation enhancer, and Cas9 protein (Alt R(TM) S.p. Cas9 Nuclease V3, 100 µg Cat# 1081058) were purchased from Integrated DNA Technologies, Inc. ((hereinafter, referred to as IDT), USA). Three types of crRNA sequences were each added at 1 µg/µl to Opti-MEMI (Thermo Fisher Scientific Inc., Cat# 31985062) and dissolved therein. The sequences of the crRNA and the electroporation enhancer are as follows.

**[Table 3]**

| Table 3: Sequence of targeting site of crRNA used in KAP8-1 disruption | | |
|---|---|---|
| Name of crRNA | Sequence (5' →3' ) | SEQ ID NO |
| KAP8-1 crRNA1 | GCAACCTGGGAAGACGGTGC | 97 |
| KAP8-1 crRNA2 | TACAGTGTGGGCTGTGGCTA | 98 |
| KAP8-1 crRNA3 | CGGTAGTACCTACTCCCCAG | 99 |
| Cas9 EP enhancer | TTAGCTCTGTTTACGTCCCAGCGGGCATGAGAGTAACAA GAGGGTGTGGTAATATTACGGTACCGAGCACTATCGATA CAATATGTGTCATACGGACACG | 100 |

### (2) Electroporation

Male and female goats were purchased from Inoue Shoten. Synchronization was started by the administration of PG (Veterinary Pronalgon F Injection(TM), Zoetis Japan) and carried out with P4 (Lutinus Vaginal Tablet 100 mg(TM), Ferring Pharmaceuticals), and follicular induction was started with E2 (Ovahormon Injection(TM), ASKA Animal Health Co., Ltd.). PG and hCG (Gestron 1500(TM), Kyoritsu Seiyaku Corp.) were administered to a female goat in which ultra-superovulation was induced by the FSH (Antrin R10(TM), Kyoritsu Seiyaku Corp.) tapering method, followed by mating with the male goat. After ovulation, the female goat was laparotomized under inhalation anesthesia to excise and recover the uterine horn and the ovary. An indwelling needle (Surflo(TM), Terumo Corp.) was put thereinto from the uterine horn side, and PBS (Embryotech(TM), Nippon Zenyaku Kogyo Co., Ltd.) was refluxed in the fallopian tube to recover *in vivo* fertilized eggs. The fertilized eggs were recovered into a culture solution (BO-HEPES-IVM(TM), IVF Bioscience) and further washed by transfer into a culture solution (BO-IVC(TM), IVF Bioscience) three times.

An electrode (BEX Co., Ltd. (hereinafter, referred to as BEX), Japan, Cat# LF501PT1-10) connected to GenomeEditor (BEX, Cat# GEB15) was loaded on a stereomicroscope. The fertilized eggs cultured in BO-IVC medium were washed twice with Opti-MEMI to remove a serum-containing medium. Subsequently, the fertilized eggs were arranged in a gap of the electrode filled with Opti-mem solution (5 µl) containing three types of crRNA (120 ng/µl) targeting KAP8-1, tracrRNA (120 ng/µl), Cas9 protein (100 ng/µl), and electroporation enhancer (150 ng/µl), and electroporated. The electrical conditions involved 20 V (3-msec pulse + 97-msec interval) three times each for one polarity and opposite polarity.

Immediately after electroporation, the fertilized eggs were recovered from the electrode and washed once with M2 medium and twice with BO-IVC medium. Subsequently, the fertilized eggs were cultured until blastocysts in BO-IVC medium at 39°C for 7 days in a 5% CO₂ and 5% O₂ incubator.

### (3) Confirmation of mutation in knockout goat

The blastocyst on culture day 7 were synchronized and transplanted into the goat uterine horn for transplantation by laparotomy under inhalation anesthesia on the same day as culture day 7. Within 1 week from delivery, a portion of the ears was collected, treated in 90 µl of 50 mM NaOH at 98°C for 10 minutes, and then neutralized with 10 µl of 1 M Tris HCl (pH 8.0) to collect a genomic DNA solution. In order to examine mutations in each gene by CRISPR/Cas9, a genomic region adjacent to each target sequence was amplified by PCR using the following specific primers.

**[Table 4]**

| Table 4: Primer for confirmation of KAP8-1 disruption | | |
|---|---|---|
| Name of primer for PCR | Sequence (5' →3' ) | SEQ ID NO |
| gKrtap8-1-Fw3 | TTGGGGTCAAGAATGAAAATGACG | 101 |
| gKAP8-1-Rv3 | AACATCATGCAGGGAAGGCA | 102 |

A PCR amplification product of each gene was purified and analyzed for the DNA sequence of a target site using commissioned sequence service provided by Eurofins Genomics K.K. (Japan).

As a result, a knockout goat having a mutated sequence of Figure 9 was obtained. In Figure 9, a wild-type sequence was compared with a mutated sequence in the knockout goat, and a sequence deleted in the knockout goat is highlighted, and the sequence of a coding region is indicated in capital letters.

KAP8-1 (heterozygous) had a mutated sequence that caused deletion of 2 amino acids (Y30 and G31) in an important region highly conserved across species, thereby disrupting the KAP8-1 gene.

In accordance with Examples described above, underlayer (undercoat) was collected from a 5-month-old goat, and a region of 10 mm in a central portion of the obtained underlayer was embedded in a resin, followed by the major axis measurement of the hair at 3 locations within the region. As a result, as shown in Figure 13, the hair of a goat of the same species before genetic modification had an average major axis of 14.55 µm, whereas the hair of the KAP8-1-disrupted goat had an average major axis of 11.46 µm, showing more than 20% decrease in major axis. This difference in average major axis was statistically significant (p < 0.01). The underlayer surface of a wild-type goat and the KAP8-1-disrupted goat was observed under an electron microscope to measure a scale width and the number of scales. The scale width was determined by measuring scale widths on upper and lower sides, respectively, of the hair in the electron microscope image of the underlayer surface, and calculating an average thereof, which was used as a scale width. The number of scales was defined as the number of scales contained in the underlayer with a given length under an electron microscope. As a result, the underlayer of the wild-type goat had a scale width of 13.2 µm, whereas the KAP8-1-disrupted goat had a scale width of 14.0 µm, which was a larger scale width. As for the number of scales, the underlayer of the KAP8-1-disrupted goat contained only 10.2 scales on average within a length containing 13.2 scales on average in the wild goat underlayer. This suggested that the disruption of KAP8-1 in a goat also decreases the number of scales contained in underlayer with a given length, i.e., results in hair that exhibits smooth texture and has high sheen.

### Example 10: Western blotting of animal fiber derived from each animal species

A protein sample was prepared from the hair of various animals, and Krt72 or KAP8-1 protein was detected by Western blot. Specifically, this operation was performed as follows.

### (1) Preparation of protein sample

The hair of each animal was cut into a length of 2 to 3 mm, washed with ethanol, and then incubated in 8 M urea, 0.1 M Tris-HCl pH 8.0, 2% SDS, and 100 mM DTT at 37°C for 1 day. The hair was further incubated at 60°C for 30 minutes and centrifuged at 10,00 rpm for 5 minutes. A supernatant was recovered, and 4 × SDS Sample buffer (FUJIFILM Wako Pure Chemical Corp.) was added in the same amount as that of the supernatant.

### (2) Western blot

Each individual protein sample (100 µg) was separated on SDS gel and electrophoretically transferred to a PVDF membrane (Millipore Corp.). The PVDF membrane with the transferred sample was dipped in Blocking One (Nacalai Tesque, Inc.) and shaken at room temperature for 1 hour for blocking treatment. Primary antibodies shown in the table below were used, diluted using Blocking One diluted 20-fold with PBST, and shaken at 4°C for 1 day. Peroxidase AffiniPure Donkey Anti-Rabbit IgG (Jackson ImmunoResearch Laboratories, Inc.) was used as a secondary antibody, diluted at 1:10000, and shaken at room temperature for 1 hour. Western ECL blotting substrate (Bio-Rad Laboratories, Inc.) was used in detection.

**[Table 5]**

| Table 5: Primary antibody in Western blotting | | | |
|---|---|---|---|
| Name of antibody | Company | Model number | Dilution |
| KRT72 Polyclonal Antibody | invitrogen | PA5-112918 | 1:2000 |
| KRTAP8-1 Antibody | novusbio | NBP1-91596 | 1:1000 |

The results were as shown in Figure 14. The animal fiber obtained from any of the animal species contained Krt72 and KAP8-1. This suggested that these hair constituents are common across species.

### Example 11: Identity of amino acid sequence of each constituent

KAP8-1 (Figure 15), Krt71 (Figure 16), Krt72 (Figure 17), and Krt74 (Figure 18) of various organism species were obtained from a database, and the identity of their amino acid sequences was determined. A region conserved among animal species was identified for each protein by motif search. This region was confirmed to be a functional domain on the basis of the NCBI database. Then, the amino acid sequence identity of this domain was calculated. As a result, all the animal species exhibited high sequence identity. Thus, the disrupted genes had high sequence identity across species, suggesting that functions of their proteins are also conserved across species.

## Claims

1. A genetically modified animal or an animal fiber thereof, wherein
the animal has the animal fiber, and
the genetic modification is reduction or deficiency in expression or functions of at least one gene constituting the Krt or KAP family, or disruption of the at least one gene.

2. The genetically modified animal or the animal fiber thereof according to claim 1, wherein
(i) the animal fiber of the genetically modified animal has a smaller major axis than that of the animal fiber of an animal that does not have the genetic modification, and/or
(ii-1) the animal fiber of the genetically modified animal has a smaller content of a protein encoded by the at least one gene than that of the animal fiber of an animal that does not have the genetic modification, or (ii-2) the animal fiber of the genetically modified animal contains no protein encoded by the at least one gene.

3. The genetically modified animal or the animal fiber thereof according to claim 1 or 2, wherein
the animal fiber of the genetically modified animal has an at least 5%, at least 10%, or at least 15% smaller major axis than that of the animal fiber of an animal that does not have the genetic modification.

4. The genetically modified animal or the animal fiber thereof according to any one of claims 1 to 3, wherein
the at least one gene constituting the Krt or KAP family at least comprises Kap8-1.

5. The genetically modified animal or the animal fiber thereof according to any one of claims 1 to 3, wherein
the at least one gene constituting the Krt or KAP family at least comprises at least one gene selected from Krt71, Krt72, and Krt74.

6. The genetically modified animal or the animal fiber thereof according to any one of claims 1 to 5, wherein
the genetic modification further comprises reduction or deficiency in expression or functions of either or both of FGF-5 gene and Tyr gene, or disruption of the gene(s).

7. The animal fiber according to any one of claims 1 to 6.

8. Cloth, textile, or a fiber product comprising the animal fiber according to claim 7.
